# EUROPÄISCHE PATENTSCHRIFT

(11) **EP 1 372 753 B1**
(45) Veröffentlichungstag und Bekanntmachung des Hinweises auf die Patenterteilung: **01.08.2007**
(21) Anmeldenummer: 02700248.4
(22) Anmeldetag: 18.02.2002
(51) Int. Cl.: A61L 31/16

(54) **IMPLANTATE MIT FK506 ZUR RESTENOSEBEHANDLUNG UND -PROPHYLAXE**
IMPLANTS WITH FK506 FOR PROPHYLAXIS AND TREATMENT OF RESTONOSES
IMPLANTS AVEC FK506 POUR LA PROPHYLAXIE ET LE TRAITEMENT DE LA RESTENOSE

(30) Priorität: 16.02.2001 DE 10107339; 05.06.2001 DE 10127011; 06.06.2001 DE 10127330
(43) Veröffentlichungstag der Anmeldung: 02.01.2004
(73) Patentinhaber: Abbott Laboratories Vascular Enterprises Limited, Terrace, Dublin 2 (IE); Astellas Pharma Inc., Chuo-ku, Tokyo (JP)
(72) Erfinder: WNENDT, Stephan, 52066 Aachen (DE); VON OEPEN, Randolf, 72074 Tübingen (DE); KUTTLER, Bernd, 72770 Reutlingen (DE); LANG, Gerhard, 72172 Sulz (DE)
(74) Vertreter: Vossius & Partner
(86) Internationale Anmeldenummer: PCT/EP2002/001707
(87) Internationale Veröffentlichungsnummer: WO 2002/065947

(56) Entgegenhaltungen:
- EP-A- 0 665 013
- EP-A- 0 970 711
- DE-C- 19 744 135

## Beschreibung

Die Erfindung betrifft Implantate, insbesondere intrakavernöse oder intravaskuläre, vorzugsweise zur Behandlung oder Prophylaxe von koronaren oder peripheren Gefäßverschlüssen oder Gefäßverengungen, insbesondere von Verengungen bzw. Stenosen oder Restenosen, vorzugsweise zur Prophylaxe von Restenose, die in chemisch kovalent oder nicht-kovalent gebundener oder physikalisch fixierter Form FK506 enthalten, Verfahren zu deren Herstellung und deren Verwendung.

Die Bildung arteriosklerotischer Lesionen in den arteriellen Blutgefäßen ist die zugrundeliegende Krankheit für eine große Bandbreite klinischer Symptome, die von Angina pectoris über Claudicatio intermittens zum Myocardinfarkt und dem ischämischen Schlaganfall reichen; alle basierend auf atheromer Bildung und/oder stenotischen Lesionen. Der Begriff stenotische Lesionen bezeichnet die lokale Reduktion des Gefäßlumens auf weniger als 60-70 % seines normalen Durchmessers, was wiederum zu einer deutlichen Reduktion der Versorgung des jeweiligen Gewebes mit Sauerstoff und Nährstoffen führt. Obgleich die Pharmakotherapie (Statine, ACE-Inhibitoren, gplla/lllb Blocker und Plasminogen-Aktivatoren) insbesondere im Bereich cardiovaskulärer Krankheiten innerhalb der letzten Jahrzehnte gute therapeutische Erfolge gezeitigt hat, sind immer noch chirurgische Eingriffe (Bypass-Operationen etc.) bei vielen Patienten notwendig, die ein komplettes ischämisches Krankheitsbild entwickelt haben. Im übrigen sind diese Operationen relativ kompliziert und kostenintensiv und beinhalten das Risiko schwerer Komplikationen.

Um das Entstehen ischämischer Herzkrankheiten zu verhindern, sind minimal invasive chirurgische Vefahren entwickelt worden. Die Erfindung perkutaner transluminaler Herzkranz-Angioplastie (Percutaneous Transluminal Coronary Angioplasty/PTCA) in den späten 70er Jahren war ein großer Durchbruch in der Cardiologie. Bei der PTCA werden aufblasbare Ballons verwendet, die bis zur stenotischen Lesion in den Koronar-Arterien vorgeschoben werden. Diese Ballons werden dann an den jeweiligen Zielpositionen aufgeblasen und erreichen eine Dilatation der stenotischen Region. Ein ähnliches Vorgehen kann auch bei der Dilatation der carotischen oder peripheren Arterien angewandt werden.

Trotzdem wurde relativ bald festgestellt, daß sich bei einem relativ großen Anteil der PTCA-Patienten an den Stellen, die mit dem Ballonkatheter dilatiert worden waren, eine wiederauftretende Stenose entwickelte. Es wurde dabei entdeckt, daß diese sogenannte Restenose durch eine Neubildung der vaskulären Architekur der Gewebeschichten entsteht. Die Einführung röhrenförmiger vaskulärer Metallimplantate, sogenannter Stents, bei der transluminalen Behandlung der Stenose verbesserte die Situation. In klinischen Studien (Serruys et al., N. Engl. J. Med. 331 (1994) 489-495) wurde nachgewiesen, daß die Anwendung von Stents an den Ballon-dilatierten Stellen das Auftreten der Restenose von ca. 45 % auf ca. 30 % zu senken vermochte. Obgleich dies bereits als signifikante Verbesserung bei der Verhinderung residualer Restenose anzusehen ist, gibt es immer noch einen deutlichen Anreiz für therapeutische Verbesserungen.

Bei detaillierten Studien der Pathophysiologie der Restenose im Stent wurde entdeckt, daß diese sich von der PTCA-induzierten Restenose unterscheidet. Entzündungsreaktionen, Hyperproliferation und die Einwanderung glatter Muskelzellen (smooth muscle cells (SMCs)) sind wichtige Faktoren der Neointima-Bildung, die zur Restenose im Stent führen. Es wurde im Tier-Restenose-Modell und selbst im menschlichen Gewebe festgestellt, daß die Hyperproliferation der SMC's mit Infiltration der Gewebe um die Aussteifungen des Stents durch Makrophagen und T-Zellen (Grewe et al., J. Am. Coll. Cardiol. 35 (2000) 157-63) einhergeht. In Analogie zu anderen klinischen Indikationen, bei denen Entzündungsreaktionen und Hyperproliferation von Zellen eine Rolle spielen und die durch medikamentöse Behandlung kontrolliert werden können, wurde auch versucht, die Restenose durch Pharmakotherapie zu behandeln. Ausgewählte Wirkstoffe wurden entweder oral oder intravenös gegeben oder durch perforierte Katheter an den Wirkort gebracht. Unglücklicherweise vermochte bis dato keiner dieser Wirkstoffe die Restenose entscheidend zu reduzieren (Gruberg et al., Exp. Opin. Invest. Drugs 9 (2000) 2555-2578).

Die direkte Abgabe von pharmakologisch aktiven Wirkstoffen von mit Wirkstoff beschichteten Stents ist hier die Methode der Wahl. Tierversuche und erste Ergebnisse klinischer Versuche mit mit Wirkstoff beschichteten Stents erwecken den Anschein, daß eine verzögerte Freisetzung immunosuppressiver oder antiproliferativer Wirkstoffe das Risiko einer Restenose senken kann. Paclitaxel, ein cytostatischer Wirkstoff und Rapamycin, ein immunosuppressiver und cytostatischer Wirkstoff wurden in Tierversuchen getestet. Beide Verbindungen inhibieren die Neointima Bildung (Herdeg et al., Semin Intervent Cardiol 3 (1998) 197-199; Hunter et al., Adv. Drug. Delivery Rev. 26 (1997) 199-207; Burke et al., J Cardiovasc Pharmacol., 33 (1999) 829-835; Gallo et al., Circulation 99 (1999) 2164-2170). Trotzdem wurde nach 6 Monaten Implantation beschichteter Stents in Schweinen mit Paclitaxel eine Aufhebung des Effekts beobachtet (Heldman, International Local Drug Delivery Meeting and Cardiovascular Course on Radiation, Geneva, Jan 25-27, 2001). Rapamycin zeigte eine gute Effektivität mit einer kompletten Aufhebung der Restenose in ersten klinischen Anwendungen (Sousa et al., Circulation 103 (2001) 192-195). Andererseits scheint dies Hand in Hand mit einer verlangsamten Heilung der durch Ballon-Angioplastie und Stent-Platzierung verletzten Gefäßwand einher zu gehen.

Allgemein gesprochen ist ein Gleichgewicht zwischen Heilung der arteriellen Gefäßwand nach Angioplastie und Stent-Platzierung auf der einen Seite und der Eindämmung der Neointima-Bildung von großer Wichtigkeit. Um dieses Gleichgewicht zu erreichen, sollten Wirkstoffe verwendet werden, die selektiv mit spezifischen Mechanismen, die zur Neointima-Bildung führen, interferieren.

EP-A-0 970 711 beschreibt ein Verfahren zum Beschichten von Stends mit polymerhaltigen Lösungen, die pharmazeutische Wirkstoff, wie, FK-506 enthalten können.

Daher war es Aufgabe der Erfindung, ein intrakavernöses, vorzugsweise ein intravaskuläres Implantat mit günstigen Eigenschaften zur Behandlung und Prophylaxe von Restenose zur Verfügung zu stellen. Diese Aufgabe wird mit den Merkmalen der Patentansprüche gelöst.

Gegenstand der Erfindung ist daher ein Implantat enthaltend in chemisch kovalent oder nicht-kovalent gebundener oder physikalisch fixierter Form FK506 sowie gegebenenfalls mindestens einen weiteren Wirkstoff.

Dabei gilt für jeden im Rahmen dieser Erfindung genannten Wirkstoff, inklusive des Wirkstoffes FK506, daß der Begriff Wirkstoff auch direkte Derivate des Wirkstoffes sowie den Wirkstoff auch in allen Arten von Salzen, Enantiomeren, Razematen, Basen oder freien Säuren des Wirkstoffes sowie Mischungen daraus beschreibt.

Dabei versteht man unter intrakavernös innerhalb eines Hohlraums, insbesondere innerhalb eines Hohlorgans bzw. von Hohlorganen wie Blutgefäßen, Speiseröhren, Harnleiter, Gallengängen etc.

Unter intravaskulär ist insbesondere der Einsatz in einem Blutgefäß zu verstehen.

Bevorzugt ist es auch, wenn das Implantat zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose geeignet ist.

Besonders bevorzugt ist daher ein intrakavernöses, bevorzugt intravaskuläres Implantat zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose, enthaltend in chemisch kovalent oder nicht-kovalent gebundener oder physikalisch fixierter Form FK506 sowie gegebenenfalls mindestens einen weiteren Wirkstoff.

Das makrolide Antibiotikum FK506 (Tacrolimus, [3S-[3R*[E(1S*, 3S*, 4S*)], 4S*, 5R*,-8S*, 9E, 12R*, 14R*, 15S*, 16R*, 18S*, 19S*, 26aR*]]-5,6,8,11,12, 13, 14, 15, 16, 17, 18, 19, 24, 25, 25, 26, 26a-Hexadecahydro-5,19-dihydroxy-3-[2-(4-hydroxy-3-methoxycyclohexyl)-1-methylenthenyl]-14,16-dimethoxy-4,10,12,18-tetramethyl-8-(2-propenyl)-15, 19-epoxy-3H-pyrido[2,1-c][1,4]oxaazacydotricosine-1,7,-20,21 (4H,23H)-tetrone; Merck index no. 9000.) ist ein für die Transplantationsmedizin entwickelter Wirkstoff. FK506 inhibiert die Abgabe von Interleukin-2 (IL-2) und Interferon-γ (IFN-γ) aus T-Zellen und blockt dabei die Abstoßung des Implantats (Grafts) (Wiederrecht et al., Ann. NY Acad Sci. 696 (1993) 9-19). FK506 wurde auch in SMC-Kulturen in Hinblick auf eine Inhibition der Proliferation der glatten Muskelzellen (Mohacsi et al., J. Heart Lung Transplant. 16 (1997) 484-492; Marx et al., Circulation Res., 76 (1995) 412-417) und ihrer Migration (Poon et al., J. Clin. Invest. 98 (1996) 2777-2283) untersucht. Generell wurde FK506 von verschiedenen Forschern wegen seiner geringen Potenz als ungeeignet für die Prävention der Restenose eingeschätzt (Mohacsi et al. (1997); Poon et al. (1996); Marx et al., 1995; Dell, Curr Med Chem 5 (1998) 179-94). Mohacsi et al. fanden eine halbmaximale Inhibition der SMC-Proliferation zwischen 100 nM and 1 µM, während Marx et al. bei Konzentrationen bis zu 123 nM gar keinen Effekt beobachteten. Im Gegensatz dazu ist Rapamycin bei der Inhibierung der Proliferation der SMC-Kulturen im nanomolaren Konzentrationsbereich aktiv.

Ausgehend von diesem Stand der Technik schien der Einsatz ausgerechnet von FK506 zur Hemmung der Restenose überhaupt nicht Erfolg versprechend (Mohacsi et al. (1997); Poon et al. (1996)). Überraschenderweise hatte es sich aber entgegen der Meinung der Fachwelt gezeigt, daß insbesondere der Einsatz von FK506 als Teil eines Stents aber auch anderer Implantate sehr effektiv in der Behandlung und Prophylaxe von Restenose ist. Gerade die lokale Applikation von FK506 ist günstig zur Verhinderung der Restenose und die Wirkung ist sehr ausbalanciert, denn sie erlaubt auch eine gute Re-Endothelialisierung der verletzten Gefäßwand.

Ohne daß dies von vorn herein anzunehmen war, könnte sich dies möglicherweise mit der immunomodulatorischen Aktivität von FK506 erklären lassen, die sich in einer halbmaximalen Inhibition der IL-2-Freisetzung bei Konzentrationen um 0,1 nM (Kino et al., J. Antibiot. 40 (1987) 1256-1265) und einem inhibitorischen Effekt auf die SMC-Proliferation bei Konzentrationen um 300-500 nM zeigt. Entsprechend günstig ist daher der Einsatz von FK506.

Dabei versteht man unter Stenose den Verschluß oder die Verengung eines Gefäßes und unter Restenose das Wiederauftreten einer Stenose.

Weiter versteht man hier unter "enthaltend" unter anderem auch eine beispielsweise nicht kovalent gebundene Beschichtung.

Weiter bezieht sich hier "peripher" insbesondere auf Gefäße bzw. andere Hohlorgane außerhalb des Herzens und der Herzkranzgefäße.

Unter "chemisch nichtkovalent" gebunden sind insbesondere Bindungen durch Wechselwirkungen wie Wasserstoffbrücken, hydrophobe Wechselwirkungen, Van der Waals-Kräfte etc. zu verstehen.

Unter physikalisch fixiert ist beispielsweise das Einschließen z.B. durch eine Membran in einem Loch zu verstehen oder sterisches Entrapment durch Wahl der Öffnungsgrößen etc..

Unter Implantat ist jede Form eines künstlichen Objektes, das (auch nur zeitlich begrenzt) eingebracht wird, zu verstehen. Insbesondere handelt es sich dabei um intrakavernöse, beispielsweise intravaskuläre Implantate. Beispiele sind Stents, Grafts, Stent Grafts, Graft-Verbinder, Führungsdrähte, Katheterpumpen oder Katheter.

Unter Stent versteht man im Sinne dieser Erfindung ein längliches, im Inneren hohles Implantat mit mindestens zwei Öffnungen und meist kreisförmigem oder eliptischem aber auch beliebigem anderen Querschnitt (meist aus Metall aber gegebenenfalls auch aus Kunststoffmaterialien bzw. Polymeren) von bevorzugt durchbrochener, gitterförmiger Struktur, das in Gefäßen, insbesondere Blutgefäßen implantiert wird, um diese offen bzw. funktionsfähig zu halten.

Unter Graft versteht man im Sinne dieser Erfindung ein längliches, im Inneren hohles Implantat mit mindestens zwei Öffnungen und meist kreisförmigem oder eliptischem aber auch beliebigem anderen Querschnitt und mit mindestens einer homogenen oder gegebenenfalls aus verschiedenen Strängen gewebten, geschlossenen und für korpuskuläre Blutbestandteile und/oder für Wasser undurchlässigen PolymerOberfläche, das im allgemeinen als Gefäß-Prothese dient und meist bei beschädigten Gefäßen oder anstatt Gefäßen eingesetzt wird.

Unter Stent Graft versteht man im Sinne dieser Erfindung die Verbindung zwischen Stent und Graft. Entsprechend ist ein Stent Graft im Grunde eine mit einem Stent verstärkte Gefäß-Prothese (Graft s.o.), wobei die Polymerschicht homogen oder gegebenenfalls aus verschiedenen Strängen gewebt, geschlossen und für korpuskuläre Blutbestandteile und/oder für Wasser undurchlässig ist. Im engeren Sinne ist dies ein Stent, der auf mindestens 20% der Oberfläche des Implantats eine durchbrochene (gitterförmige), bevorzugt metallene Außenschicht und mindestens eine innerhalb oder außerhalb dieser Außenschicht liegende homogene oder gegebenenfalls aus verschiedenen Strängen gewebte, geschlossene und für korpuskuläre Blutbestandteile und/oder für Wasser undurchlässige Polymerschicht sowie gegebenenfalls (bei außenliegender durchbrochener Schicht) eine weitere innerhalb der Polymerschicht liegende, durchbrochene (gitterförmige) bevorzugt metallene Innenschicht oder eine außen und außerhalb der durchbrochenen Schicht liegende homogene oder gegebenenfalls aus verschiedenen Strängen gewebte, geschlossene und für korpuskuläre Blutbestandteile und/oder für Wasser undurchlässige Polymerschicht aufweist.

Unter Graftverbinder versteht man im Sinne dieser Erfindung ein Implantat, daß mindestens zwei Hohlorgane, Gefäße oder Grafts verbindet, aus den für Grafts oder Stent Grafts definierten Materialien besteht und/oder den für diese definierten Aufbau hat und entsprechend mindestens zwei, vorzugsweise drei oder vier Öffnungen aufweist, insbesondere eine asymetrische "T"-Form zeigt.

Unter Katheter versteht man im Sinne dieser Erfindung ein röhrenförmiges Instrument zur Einführung in Hohlorgane. Im engeren, bevorzugten Sinne sind es Führungs-, Angiographie- oder Ballonkatheter.

Unter Katheterpumpe versteht man im Sinne dieser Erfindung einen an seiner Spitze mit einem Propeller versehenen Katheter, der das Pumpen des Herzmuskels unterstützen kann.

Es ist eine weitere bevorzugte Ausführungsform des erfindungsgemäßen Implantats, wenn das Implantat wenigstens eine aus einem Metall oder einer Metalllegierung bestehende, homogene oder aus verschiedenen Strängen gebildete, geschlossene oder durchbrochene Schicht oder Oberfläche aufweist.

Unter Metall bzw. Metall-Legierung im Sinne dieser Erfindung sind insbesondere Stahl bzw. Stahl-Legierungen aber auch Nickel bzw. Nickel-Legierungen zu verstehen, wobei der Begriff Metall bereits von vorneherein auch Metall-Legierungen mitumfaßt.

Unter durchbrochen sind insbesondere gitterförmige, gewebte oder geflochtene Strukturen zu verstehen.

Es ist eine weitere bevorzugte Ausführungsform des erfindungsgemäßen Implantats, wenn das Implantat wenigstens eine aus einem Polymer bestehende, homogene oder aus verschiedenen Strängen gebildete, geschlossene oder durchbrochene Schicht oder Oberfläche aufweist.

Bei einer bevorzugten Ausführungsform weist das Implantat wenigstens eine Polymerschicht auf, die ganz oder teilweise eine aus einem Metall oder einer Metalllegierung bestehende, homogene oder aus verschiedenen Strängen gebildete, geschlossene oder durchbrochene Schicht oder Oberfläche, vorzugsweise eine aus einem Metall oder einer Metalllegierung bestehende, gegebenenfalls gitterförmige Struktur, bedeckt.

Bei einer besonders bevorzugten Ausführungsform weist das Implantat wenigstens eine aus einem Metall oder einer Metalllegierung bestehende, homogene oder aus verschiedenen Strängen gebildete, geschlossene oder durchbrochene Schicht oder Oberfläche und wenigstens eine aus einem Polymer bestehende, homogene oder aus verschiedenen Strängen gebildete, geschlossene oder durchbrochene Schicht oder Oberfläche auf.

Dabei ist es besonders bevorzugt, wenn bei diesem Implantat die aus einem Metall oder einer Metalllegierung bestehende Schicht oder Oberfläche eine aus einem Metall oder einer Metalllegierung bestehende, gegebenenfalls gitterförmige Struktur ist und/oder die aus einem Polymer bestehende Schicht oder Oberfläche homogen geschlossen oder gewebt ist und/oder wasser- und/oder korpuskelundurchlässig ist und/oder die Abfolge der Schichten und Oberflächen von außen nach innen Metall-Polymer, Polymer-Metall, Metall-Polymer-Metall oder Polymer-Metall-Polymer ist und/oder entweder die aus einem Polymer bestehende Schicht oder Oberfläche nicht chemisch (kovalent oder nichtkovalent) mit der aus einem Metall oder einer Metalllegierung bestehenden Schicht oder Oberfläche verbunden ist oder die aus einem Polymer bestehende Schicht oder Oberfläche mittels eines Klebstoffes mit der aus einem Metall oder einer Metalllegierung bestehenden Schicht oder Oberfläche verbunden ist.

Es ist weiter bevorzugt, wenn das im Rahmen der Implantate verwendete Polymer ausgewählt ist aus Dacron; Polytetrafluorethylen (PTFE/Teflon), ausdehnbar oder nicht ausdehnbar; oder Polyurethan; vorzugsweise aus Polytetrafluorethylen (PTFE), ausdehnbar oder nicht ausdehnbar; oder Polyurethan; insbesondere aus PTFE.

Es ist auch eine bevorzugte Ausführungsform der Erfindung, wenn das Implantat ein Stent, ein Stent Graft, ein Graft, ein Graft-Verbinder, ein Führungsdraht, ein Katheter oder eine Katheterpumpe, vorzugsweise ein Stent, ein Stent Graft, ein Graft oder ein Graft-Verbinder, insbesondere ein Stent oder ein Stent Graft ist.

Es ist besonders bevorzugt, wenn das erfindungsgemäße Implantat mit FK506 beschichtet ist.

Eine lokale Anwendung von FK506 wird durch direkte Abgabe von der wirkstoffbeladenen Oberfläche eines koronaren oder peripheren Stents erreicht. Eine wirkstoffbeladene Oberfläche eines Stents kann durch Verwendung verschiedener technologischer Ansätze erreicht werden. Jeder dieser Ansätze kann so durchgeführt werden, daß der Wirkstoff von der Oberfläche entweder in einem kurzen (Stunden) oder einem ausgedehnten Zeitraum (Tage) freigesetzt wird. Die Freisetzungskinetik kann dadurch angepaßt werden, daß spezifische Modifikationen an der Oberfläche vorgenommen werden, z.B. hydrophobe oder hydrophile Seitenketten eines polymeren Trägers oder eine keramische Oberfläche. Auch diese Oberflächen können ihrerseits wieder an der Oberfläche modifiziert werden, z.b. durch Si-Gruppen auf einer Aluminiumoxid-Schicht.

Die Freisetzungskinetik kann dadurch angepasst werden, dass spezifische Polymere, Blockpolymere, Polymerblends, Pfropfpolymere alleine oder im Schichtaufbau eingesetzt werden. Besonders geeignet zur Steuerung der Freisetzungskinetik ist die Verwendung von Nanokapseln und/oder Liposomen und den oben beschriebenen Polymerkombinationen. Unter Nanokapseln versteht man im allgemeinen das Umhüllen von mizellaren Systemen oder kolloidalen Feststoffen zu ultrafeinen Partikeln mit einem festen Überzug. Die umhüllten Teilchen, deren Größe im Nanometerbereich liegt, lösen sich kolloidal. Nanoverkapselte Wirkstoffe können so mit verlängerter Wirksamkeit eingesetzt werden. Liposomen werden im allgemeinen aus Phospholipiden durch Dispergieren in wäßrigen Medien gebildet und sind in diesem Zusammenhang interessant, da hydrophile Wirkstoffe in das wäßrige Innenvolumen wie auch in die wäßrigen Zwischenschichten und hydrophobe Wirkstoffe in die Lipidschichten eingebaut werden können. Verwendet man Nanokapseln und/oder Liposomen in unterschiedlicher Zusammensetzung so können diese mit verschiedenen Wirkstoffen beladen werden und so eine Wirkstoffkombination gezielt freigesetzt werden.

### Keramische Beschichtung

Eine Aluminiumoxid-Beschichtung (Patentanmeldungen DE 19855421, DE 19910188, WO 00/25841) mit poröser Oberfläche kann mit FK506 mit Mengen zwischen 10 µg und 10 mg entweder durch Eintauchen, Aufsprühen oder einer vergleichbaren Technik beladen werden. Die Dosis des Wirkstoffes hängt von der Art des Zielgefäßes und dem Zustand des Patienten ab und wird so gewählt, daß Proliferation, Migration und T-Zell-Antwort ausreichend inhibiert werden, ohne daß der Heilungsprozeß behindert wird. FK506 kann als wässrige oder organische Lösung, beispielsweise in DMSO, DMF und Ethanol verwendet werden. Nach dem Sprühen oder Eintauchen (gegebenfalls unter schwachem Vakuum) wird der behandelte Stent getrocknet und der Vorgang 2-10-mal wiederholt. Eine andere Möglichkeit in der Aufbringung besteht in der direkten Aufgabe der Wirkstofflösung auf die Stent-Stränge mit Hilfe einer Mikropipette oder eines Pipettierroboters. Nach dem letzten Trockenschritt kann der Stent eine Minute lang bei Raumtemperatur in Wasser oder isotonischer Saline gespült und anschließend wieder getrocknet werden. Der Wirkstoffgehalt kann nach Herauslösen des Wirkstoffs mit einem geeigneten Lösungsmittel durch Standardmethoden (HPLC, LC-MS) analysiert werden. Freisetzungskinetiken können unter Verwendung einer Standard-Freisetzungsmeßapparatur gemessen werden. Der keramische Ansatz kann mit einer polymeren (gegebenenfalls bioabbaubaren) Beschichtung kombiniert werden.

Im Übrigen kann jede Art von Metalloxid-Beschichtungen analog eingesetzt werden, insbesondere zum Beispiel Iridiumoxid wie im Patent US6,245,104 B1 beschrieben. Entsprechend ist jede Nennung von Aluminiumoxid im Folgenden so zu verstehen, daß darunter auch andere Metalloxide wie zum Beispiel Iridiumoxid zu verstehen sind.

### PTFE Membran: Stent Graft

Hier wird ein zu oben beschrieben vergleichbarer Ansatz verwendet. FK506 wird in den Vertiefungen der porösen PTFE-Membran eingelagert.

### Allgemeine polymerische Beschichtung

Verschiedene Polymere sind für eine Wirkstoffbeladung geeignet: Methacrylate-Polymere, Polyurethan-Beschichtungen, PTFE-Beschichtungen, Hydrogel-Beschichtungen. Der Wirkstoff kann entweder auf die End-Oberfläche aufgetragen werden (s.o.) oder wird direkt der Polymerisationslösung beigegeben. In den übrigen Details entspricht dieser technische Ansatz den oben bereits beschriebenen.

Verschiedene Polymere sowie Kombinationen dieser in Form von Polymerblends, Systeme mit Schichtaufbau, Blockcopolymer, Pfropfpolymere können verwendet werden. Geeignete Polymere sind: Acrylate- und Methacrylate, Silikone wie z.B. Polydimethylsiloxan, Polymethylenmalonester, Polyether, Polyester, bioresorbierbare Polymere, Polymere aus vinylischen Monomeren wie z.B. Polyvinylpyrrolidon und Vinylether, Poly-cis-1,4-butadien, Poly-cis-1,4-isopren, Poly-trans-1,4-isopren sowie vulkanisierte Produkte, Polyurethane, Polyharnstoffe, Polyamide, Polyimide, Polysulfone sowie Biopolymere wie z.B. Cellulose und ihre Derivate und Proteine und Fibrinkleber. Besonders interessante Eigenschaften zeigen Hydrogele welche Wegen ihrer hohen Wasseraufnahme als äußerste Schicht (top coat) sehr gute Hämokompatibilität aufweisen. Hier können Hydrolgele wie z.B Polyacrylamid, Polyacrylsäure, Polymere mit Sauerstoff als Heteroatom in der Hauptkette wie z.B. Polyethylenoxid, Polypropylenoxid, Polytetrahydrofuran eingesetzt werden. Der Wirkstoff kann entweder auf die End-Oberfläche aufgetragen werden oder eingebettet in Nanokapseln und/oder Liposomen appliziert werden. Der Wirkstoff kann aber auch direkt in der Polymerisationslösung oder in der Polymerlösung enthalten sein. Bei einigen Polymer/Wirkstoffsystemen kann der Wirkstoff durch Quellen immobilisiert werden.

### Mechanischer Ansatz

Der mechanische Ansatz beruht auf Vertiefungen, die in die Stent-Streben mittels eines Lasers eingebracht werden. Diese Vertiefungen können dann mit FK506 gefüllt werden. Der mechanische (Vertiefungen)-Ansatz kann mit einer polymeren, gegebenenfalls bioabbaubaren Beschichtung kombiniert werden, die selbst wirkstoffbeladen ist. Nach einer anfänglichen Freisetzung aus der polymeren Beschichtung kann aus den wirkstoffgefüllten Vertiefungen langfristig Wirkstoff freigesetzt werden. In den übrigen Details entspricht dieser technische Ansatz den oben bereits beschriebenen.

Entsprechend ist es eine weitere bevorzugte Ausführungsform des erfindungsgemäßen Implantats, wenn das Implantat einen keramischen Überzug, insbesondere aus Aluminiumoxid, aufweist, an den FK506 gebunden ist.

Es ist eine weitere bevorzugte Ausführungsform des erfindungsgemäßen Implantats, wenn das Implantat einen polymeren Überzug, insbesondere aus Methacrylat-Polymeren, Polyurethan, PTFE, Hydrogel oder Hydrogel/Polyurethan-Blend, insbesondere PTFE aufweist, an den FK506 gebunden ist oder in dem FK506 vor Aufbringung des Überzugs gelöst wurde.

Es ist eine weitere bevorzugte Ausführungsform des erfindungsgemäßen Implantats, wenn das Metall des Implantats mittels Laser eingebrachte Vertiefungen aufweist, die mit FK506 gefüllt sind. Dabei ist es besonders günstig, wenn das mit FK506-gefüllten Vertiefungen versehene Metall oder mindestens die Vertiefungen mit einem biologisch abbaubaren Polymermaterial überzogen wird/werden, wobei gegebenenfalls FK506 an den polymeren Überzug gebunden wird oder FK506 vor der Polymerisation des Überzugs in dem Polymermaterial gelöst wurde.

Bei einer anderen sehr günstigen Ausführungsform des erfindungsgemäßen Implantats ist das Implantat herstellbar durch ein Verfahren, bei dem
a) ein wenigstens eine aus einem Metall oder einer Metall-Legierung bestehende, homogene oder aus verschiedenen Strängen gebildete, geschlossene oder .durchbrochene Schicht oder Oberfläche aufweisendes Substrat, das mit einem keramischen Überzug, insbesondere aus Aluminiumoxid, überzogen wird, eingesetzt wird
   oder
b) ein wenigstens eine aus einem Polymer bestehende, homogene oder aus verschiedenen Strängen gebildete, geschlossene oder durchbrochene Schicht oder Oberfläche aufweisendes Substrat eingesetzt wird,
   oder
c) ein Substrat, das mit einem polymerisierten oder auf der Oberfläche polymerisierenden Überzug, insbesondere aus Methacrylat-Polymeren, Polyurethan, PTFE, Hydrogel oder Hydrogel/Polyurethan-Blend, überzogen wird, eingesetzt wird
   oder
d) ein Substrat mit wenigstens einer aus einem Metall oder einer Metall-Legierung bestehenden, homogenen oder aus verschiedenen Strängen gebildeten, geschlossenen oder durchbrochenen Schicht oder Oberfläche, in die mittels eines Lasers Vertiefungen eingebracht werden, die mit FK506 gefüllt werden, und dann das Substrat mit einem polymerisierten oder auf der Oberfläche polymerisierenden bioabbaubaren Überzug überzogen wird, eingesetzt wird,
e) dann das Substrat gemäß a), b), c) oder d) mit einer FK506-Lösung in wäßrigem oder organischem Lösungsmittel in Kontakt gebracht wird, beispielsweise durch Beträufeln, Besprühen oder Eintauchen gegebenenfalls unter Vakuum,
f) dann gegebenenfalls das so behandelte Substrat getrocknet wird, vorzugsweise bis zur Entfernung des Lösungsmittels aus Schritt e),
g) dann gegebenenfalls Schritt e) gegebenenfalls gefolgt von Schritt f) wiederholt wird, vorzugsweise mehrfach, insbesondere 1 bis 5-fach, sowie
h) gegebenenfalls anschließen das so behandelte Substrat ein- oder mehrfach mit Wasser oder isotoner Saline gespült wird und
i) gegebenenfalls anschließend getrocknet wird.

Dabei ist bevorzugt, wenn bei der Herstellung dieses so herstellbaren erfindungsgemäßen implantats in Schritt e) FK506 in Alkohol gelöst wird, vorzugsweise in Ethanol, insbesondere in einer Konzentration von 0,5 - 5 g/l in FK506 in Ethanol und/oder in Schritt e) das Implantat unter Vakuum vorzugsweise über Nacht durch Eintauchen mit einer FK506-Lösung in wäßrigem oder organischem Lösungsmittel in Kontakt gebracht wird und/oder die Schritte f) und/oder g) nicht ausgeführt werden und/oder bei Schritt h) das Implantat mehrfach mit Saline gewaschen wird und/oder in Schritt i) das Implantat über Nacht getrocknet wird.

In einer alternativen besonders bevorzugten Ausführungsform der Erfindung ist bevorzugt, wenn bei der Herstellung dieses wie oben beschrieben herstellbaren erfindungsgemäßen Implantats in Schritt e) das Implantat, vorzugsweise steril, in ein vorzugsweise steriles Gefäß mit einem sich nach Ende einer Perforation verschließenden, perforierbarem Verschluß, beispielsweise in eine Injektions-Flasche, eingebracht wird, FK506-Lösung, vorzugsweise steril, in das Gefäß gefüllt wird, dieses mit dem sich nach Ende einer Perforation verschließenden, perforierbarem Verschluß verschlossen wird, ein dünnes, vorzugsweise steril, luftdurchlässiges Belüftungsrohr, beispielsweise eine Kanüle, perforierend durch den Verschluß gesteckt wird, ein Vakuum angelegt und vorzugsweise die FK506-Lösung bewegt wird und abschließend, vorzugsweise nach Ablauf von ca. 12 h, das dünne, vorzugsweise steril, luftdurchlässige Belüftungsrohr entfernt wird und/oder in Schritt e) FK506 in Alkohol gelöst wird, vorzugsweise in Ethanol, insbesondere in einer Konzentration von 3,3 mg FK506 in 1 ml Ethanol und/oder daß das Implantat bis zum Einsatz im vorzugsweise sterilen verschlossenen Glasgefäß von Schritt e) verbleibt und/oder die Schritte f) bis i) unterbleiben.

Bei einer anderen sehr günstigen Ausführungsform des erfindungsgemäßen Implantats ist das Implantat herstellbar durch ein Verfahren, bei dem vor Bildung wenigstens einer aus einem Polymer bestehenden, geschlossenen oder durchbrochenen Schicht oder Oberfläche oder eines polymeren Überzugs des Implantats FK506 im Polymerisations-Material gelöst wurde.

Es ist weiter besonders bevorzugt, wenn nach Implantierung des erfindungsgemäßen Implantats FK506 freigesetzt wird. Dabei ist besonders günstig, wenn die Freisetzung verzögert erfolgt. Dabei ist es eine besonders bevorzugte Ausführungsform der Erfindung, wenn FK506 vom Implantat über einen Zeitraum von 24 h, vorzugsweise 48h, insbesondere mehr als 96h nach Implantierung freigesetzt wird. Insbesondere ist auch günstig, wenn das FK506 vom Implantat nach Implantation
a) innerhalb von < 48 h oder
b) über mindestens 48 h, vorzugsweise über mindestens 7 Tage, insbesondere über mindestens 2 bis zu 21 Tagen freigesetzt wird, oder daß
c) das Implantat beide Freisetzungsmuster a) und b) zeigt.

Gerade letztere Variante ist dann zu erreichen, wenn man zwei verschiedene Arten der Beschichtung, Bindung oder physikalischen Fixierung wählt. Ein Beispiel sind die mit FK506-beladenen bioabbaubaren Membranen versiegelten Laservertiefungen mit FK506. Nach rascher Freisetzung aus der Membran wird langfristig aus den Vertiefungen freigesetzt.

Es ist eine weitere bevorzugte Ausführungsform der Erfindung, wenn im Implantat mindestens ein weiterer Wirkstoff enthalten ist, vorzugsweise ein pharmazeutischer Wirkstoff, insbesondere einen weiteren Wirkstoff ausgewählt aus den folgenden Wirkstoffen bzw. deren Derivaten
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1H-pyrazolo[3,4-n]pyridin-3-yl]-pyrimidin-4-ylamine); Hydralazin; Verapamil, Diltiazem, Nifedipin, Nimodipin oder anderen Ca²⁺-Kanal-Blockern; Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder anderen Corticosterioden; 17-beta-Östradiol; Cyclosporin; Mycophenol-Säure; VEGF, VEGF-Rezeptor Aktivatoren; Tranilast; Meloxicam, Celebrex, Vioxx oder anderen COX-2 Antagonisten; Indomethacin, Diclofenac, Ibuprofen, Naproxen oder anderen COX-1-Inhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK oder Interleukin-10;
(Gruppe 3:) Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavopiridol; Actinomycin D; Rheopro/Abciximab oder Probucol.

Dabei ist es besonders bevorzugt, daß wenn der weitere Wirkstoff ausgewählt ist aus Gruppe 1, dieser vom Implantat innerhalb der ersten 24 - 72 h nach Implantation freigesetzt wird und/oder, wenn der weitere Wirkstoff ausgewählt ist aus Gruppe 2, dieser vom Implantat innerhalb der ersten 48 h - 21 Tagen nach Implantation freigesetzt wird und/oder, wenn der weitere Wirkstoff ausgewählt ist aus Gruppe 3, dieser vom Implantat innerhalb von 14 Tagen bis 3 Monaten nach Implantation freigesetzt wird.

Ein weiterer Gegenstand der Erfindung ist ein Verfahren zur Herstellung eines erfindungsgemäßen Implantats, bei dem vor Bildung wenigstens einer aus einem Polymer bestehenden, geschlossenen oder durchbrochenen Schicht oder Oberfläche oder eines polymeren Überzugs des Implantats FK506 im Polymerisations-Material gelöst wurde.

Ein weiter Gegenstand der Erfindung ist ein Verfahren zur Herstellung eines erfindungsgemäßen Implantats mit folgenden Schritten:
a) ein wenigstens eine aus einem Metall oder einer Metall-Legierung bestehende, homogene oder aus verschiedenen Strängen gebildete, geschlossene oder durchbrochene Schicht oder Oberfläche aufweisendes Implantat gemäß einem der Ansprüche 4, 6 oder 7 bis 10, das mit einem keramischen Überzug, insbesondere aus Aluminiumoxid, überzogen wird,
   oder
b) ein wenigstens eine aus einem Polymer bestehende, homogene oder aus verschiedenen Strängen gebildete, geschlossene oder durchbrochene Schicht oder Oberfläche aufweisendes Implantat gemäß einem der Ansprüche 3 bis 8,
   oder
c) ein Implantat gemäß einem der Ansprüche 1 bis 8, das mit einem polymerisierten oder auf der Oberfläche polymerisierenden Überzug, insbesondere aus Methacrylat-Polymeren, Polyurethan, PTFE, Hydrogel oder Hydrogel/Polyurethan-Blend, überzogen wird,
   oder
d) ein Implantat gemäß einem der Ansprüche 1,2 oder 4 bis 8 mit wenigstens einer aus einem Metall oder einer Metall-Legierung bestehenden, homogenen oder aus verschiedenen Strängen gebildeten, geschlossenen oder durchbrochenen Schicht oder Oberfläche, in die mittels eines Lasers Vertiefungen eingebracht werden, die mit FK506 gefüllt werden, und dann das Implantat mit einem polymerisierten oder auf der Oberfläche polymerisierenden vorzugsweise bioabbaubaren Überzug überzogen wird,
   wird eingesetzt,
e) dann wird das Implantat gemäß a), b), c) oder d) mit einer FK506-Lösung in wäßrigem oder organischem Lösungsmittel in Kontakt gebracht, beispielsweise durch Beträufeln, Sprühen oder Eintauchen, gegebenenfalls unter Vakuum,
f) dann wird gegebenenfalls das Implantat getrocknet, vorzugsweise bis das Lösungsmittel aus Schritt e) verdampft ist,
g) dann wird gegebenenfalls Schritt e) gegebenenfalls gefolgt von Schritt f), vorzugsweise mehrfach, insbesondere 1 bis 5-fach, wiederholt sowie
h) gegebenenfalls wird anschließend das Implantat ein- oder mehrfach mit Wasser oder isotoner Saline gespült und
i) gegebenenfalls wird anschließend getrocknet.

Besonders bevorzugt ist dieses Verfahren, wenn in Schritt e) FK506 in Alkohol gelöst wird, vorzugsweise in Ethanol, insbesondere in einer Konzentration von 0,5 - 5 g/l FK506 in Ethanol und/oder daß in Schritt e) das Implantat unter Vakuum vorzugsweise über Nacht durch Eintauchen mit einer FK506-Lösung in wäßrigem oder organischem Lösungsmittel in Kontakt gebracht wird und/oder daß die Schritte f) und/oder g) nicht ausgeführt werden und/oder daß bei Schritt h) das Implantat mehrfach mit Saline gewaschen wird und/oder daß in Schritt i) das Implantat über Nacht getrocknet wird.

Eine besonders bevorzugte Alternative des erfindungsgemäßen Verfahrens liegt vor, wenn in Schritt e) das Implantat, vorzugsweise steril, in ein vorzugsweise steriles Gefäß mit einem sich nach Ende einer Perforation verschließenden, perforierbarem Verschluß, beispielsweise in eine Injektions-Flasche, eingebracht wird, FK506-Lösung, vorzugsweise steril, in das Gefäß gefüllt wird, dieses mit dem sich nach Ende einer Perforation verschließenden, perforierbarem Verschluß verschlossen wird, ein dünnes, vorzugsweise steril, luftdurchlässiges Belüftungsrohr, beispielsweise eine Kanüle, perforierend durch den Verschluß gesteckt wird, ein Vakuum angelegt und vorzugsweise die FK506-Lösung in Bewegung gehalten wird und abschließend vorzugsweise nach Ablauf von ca. 12h das dünne, vorzugsweise steril, luftdurchlässige Belüftungsrohr entfernt wird und/oder daß in Schritt e) FK506 in Alkohol gelöst wird, vorzugsweise in Ethanol, insbesondere in einer Konzentration von 3,3 mg FK506 in 1 ml Ethanol und/oder daß das Implantat bis zum Einsatz im sterilen verschlossenen Glasgefäß von Schritt e) verbleibt und/oder daß die Schritte f) bis i) unterbleiben.

Diese Verfahrensalternative ist besonders günstig, bisher völlig unbekannt und sowohl in den Kosten, als auch Herstellungszeit und -schritten äußerst vorteilhaft, zumal das Implantat sofort bereits steril verpackt entsteht. Es ist auch generell anwendbar und natürlich nicht auf FK506 beschränkt sondern funktioniert mit einer großen Vielzahl von Wirkstoffen. Gerade an solchen günstigen und einfachen Verfahren herrscht aber Mangel, so daß hier eine Aufgabe liegt.

Ein weiterer separater Gegenstand der Anmeldung ist daher ein im folgenden generelles genanntes Verfahren zur Herstellung mit Wirkstoffen beschichteter Implantate mit folgenden Schritten:
a) das Implantat wird, vorzugsweise steril, in ein vorzugsweise steriles Gefäß mit einem sich nach Ende einer Perforation verschließenden, perforierbarem Verschluß, beispielsweise in eine Injektions-Flasche eingebracht
b) vorzugsweise sterile Wirkstofflösung, vorzugsweise in einem organischen Lösungsmittel mit geringem Dampfdruck, insbesondere in Alkohol wie Ethanol oder Methanol, wird in das Gefäß eingebracht.
c) das Gefäß wird mit dem sich nach Ende einer Perforation verschließenden, perforierbarem Verschluß verschlossen,
d) ein dünnes, vorzugsweise steril, luftdurchlässiges Belüftungsrohr, beispielsweise eine Kanüle, wird perforierend durch den Verschluß gesteckt wird,
e) gegebenenfalls wird ein Vakuum angelegt, wobei vorzugsweise die Wirkstoff-Lösung bewegt wird,
f) abschließend, vorzugsweise nach Ablauf von ca. 12h, wird das dünne, vorzugsweise steril, luftdurchlässige Belüftungsrohr entfernt und
g) gegebenenfalls das Implantat bis zum Einsatz im vorzugsweise sterilen verschlossenen Glasgefäß von Schritt a) belassen.

Dabei ist für dieses generelle Verfahren günstig, wenn das Implantat von Schritt a) mindestens eine metallische durchbrochene oder geschlossene Oberfläche oder Schicht aufweist, eine keramische Beschichtung aufweist, eine polymere Beschichtung aufweist und/oder mindestens eine polymere, durchbrochene oder geschlossene Oberfläche oder Schicht aufweist.

Ebenso bevorzugt ist das generelle Verfahren für ein Implantat, das ein Stent, Stent Graft, Graft, Graft-Verbinder, Polymeroberflächen-Stent oder Katheter ist.

Bevorzugt ist das generelle Verfahren weiter, wenn der Wirkstoff ausgewählt ist aus pharmazeutischen Wirkstoffen wie beispielsweise Immunsuppressiva oder Antibiotika, vorzugsweise ausgewählt ist aus den folgenden Wirkstoffen bzw. deren Derivaten
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1 H-pyrazolo[3,4-n]pyridin-3-yl]-pyrimidin-4-ylamine); Hydralazin; Verapamil, Diltiazem, Nifedipin, Nimodipin oder anderen Ca²⁺-Kanal-Blockern; Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder Corticosterioden; FK506 (Tacrolimus); 17-beta-Östradiol; Cyclosporin; Mycophenol-Säure; VEGF, VEGF-Rezeptor Aktivatoren; Tranilast; Meloxicam, Celebrex, Vioxx oder anderen COX-2 Antagonisten; Indomethacin, Diclofenac, Ibuprofen, Naproxen oder anderen COX-1-Inhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK; Interleukin-10;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavopiridol;
Actinomycin D; Rheopro/Abciximab oder Probucol

insbesondere ausgewählt ist aus
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1 H-pyrazolo[3,4-n]pyridin-3-yl]-pyrimidin-4-ylamine); Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder Corticosterioden; FK 506 (Tacrolimus); VEGF, VEGF-Rezeptor Aktivatoren; Inhibitoren des Plasminogen Aktivators-1 (plasminogen activator inhibitors-1) oder Serpinen;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Mitoxantron; Combretastatin A4; Flavopiridol.

Ein weiterer Gegenstand der Erfindung ist die Verwendung eines erfindungsgemäßen Implantats zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose.

Ein weiterer wichtiger Gegenstand der Anmeldung ist die im folgenden FK506-Verwendung genannte Verwendung von FK506 zur Beschichtung oder zur Herstellung eines Implantats zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose.

Bevorzugt ist es für die FK506-Verwendung, wenn das Implantat ein Stent, ein Stent Graft, ein Graft, ein Graft-Verbinder, ein Führungsdraht, ein Katheter oder eine Katheterpumpe, vorzugsweise ein Stent, ein Stent Graft, ein Graft oder ein Graft-Verbinder, insbesondere ein Stent oder ein Stent Graft bzw. ein Polymeroberflächen-Stent ist.

Weiter ist es für die FK506-Verwendungen günstig, wenn das FK506 am Implantat so gebunden oder angebracht ist, daß es nach Implantierung vom Implantat, vorzugsweise verzögert, freigesetzt wird.

Ein weiterer separater Gegenstand der Anmeldung ist die Verwendung von FK506 zur Behandlung oder Prophylaxe von koronaren oder peripheren Gefäßvetengungen oder -verschlüssen, insbesondere von Verengungen bzw. Stenosen oder Restenosen, vorzugsweise zur Prophylaxe von Restenose. Wie oben ausgeführt hat hier FK506, wie im Rahmen dieser Anmeldung herausgefunden wurde, besonders günstige Eigenschaften.

Im Rahmen der Erfindung hat sich für FK506 besonders ein eine Polymerschicht aufweisender oder aus Polymeren bestehender Stent beziehungsweise ein Graft oder ein Stent Graft bewährt. Derartige Implantate, im Rahmen dieser Erfindung zusammenfassend als Polymeroberflächen-Stents bezeichnet, sind bisher noch nicht mit Wirkstoff-Beschichtung angewandt worden. Aber gerade dazu eignen sie sich ausweislich der im Rahmen dieser Anmeldung gemachten Untersuchungen hervorragend, da sie leicht mit Wirkstoff zu beladen sind und dann den Wirkstoff gleichmäßig und effektiv wieder abgeben. Diese Eigenschaft ist aber nicht auf FK506 beschränkt, so daß ein weiterer separater Gegenstand der Anmeldung ein Polymeroberflächen-Stent enthaltend in chemisch kovalent oder nicht-kovalent gebundener oder physikalisch fixierter Form mindestens einen physiologisch und/oder pharmazeutisch wirksamen Wirkstoff ist. Im Sinne dieser Erfindung versteht man unter Polymeroberflächen-Stent ein intravaskuläres Implantat im Sinne der Erfindung, das eine Polymeroberfläche aufweist. Im weiteren Sinne umfassen Polymeroberflächeh-Stents damit Grafts sowie Stent Grafts, Graft-Verbinder, mit Polymeren beschichtete oder aus Polymeren bestehende Stents, im engeren Sinne mit Polymeren beschichtete oder aus Polymeren bestehende Stents, sowie Stent Grafts.

Für den Polymeroberflächen-Stent ist es bevorzugt, wenn der Wirkstoff ausgewählt ist aus pharmazeutischen Wirkstoffen wie beispielsweise Immunsuppressiva oder Antibiotika, vorzugsweise ausgewählt ist aus den folgenden Wirkstoffen bzw. deren Derivaten
(Gruppe 1:) Moisidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1 H-pyrazolo[3,4-n]pyridin-3-yl]-pyrimidin-4-ylamine); Hydralazin; Verapamil, Diltiazem, Nifedipin, Nimodipin oder anderen Ca²⁺-Kanal-Blockem; Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder Corticosterioden; FK 506 (Tacrolimus); 17-beta-Östradiol; Cyclosporin; Mycophenol-Säure; VEGF, VEGF-Rezeptor Aktivatoren; Tranilast; Meloxicam, Celebrex, Vioxx oder anderen COX-2 Antagonisten; Indomethacin, Diclofenac, Ibuprofen, Naproxen oder anderen COX-1-Inhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK; Interleukin-10;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavopiridol;
Actinomycin D; Rheopro/Abciximab oder Probucol

insbesondere ausgewählt ist aus
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1 H-pyrazolo[3,4-n]pyridin-3-yl]-pyrimidin-4-ylamine); Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednison oder Corticosterioden; FK 506 (Tacrolimus); VEGF, VEGF-Rezeptor Aktivatoren; Inhibitoren des Plasminogen Aktivators-1 (plasminogen activator inhibitors-1) oder Serpinen;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Mitoxantron; Combretastatin A4; Flavopiridol;
und/oder daß der Polymeroberflächen-Stent mindestens zwei, vorzugsweise 2 oder 3 physiologisch und/oder pharmazeutisch wirksame Wirkstoffe ausgewählt aus einer der Gruppen 1 bis 3 enthält, vorzugsweise maximal einen Wirkstoff aus einer Gruppe.

Für eine weitere bevorzugte Ausführungsform dieses Polymeroberflächen-Stents gilt, daß, wenn der weitere Wirkstoff ausgewählt ist aus voranstehender Gruppe 1, dieser vom Implantat innerhalb der ersten 24 - 72 h nach Implantation freigesetzt wird und/oder, wenn der weitere Wirkstoff ausgewählt ist aus voranstehender Gruppe 2, dieser vom Implantat innerhalb der ersten 48 h - 21 Tagen nach Implantation freigesetzt wird und/oder, wenn der weitere Wirkstoff ausgewählt ist aus voranstehender Gruppe 3, dieser vom Implantat innerhalb von 14 Tagen bis 3 Monaten nach Implantation freigesetzt wird.

Generell gilt für erfindungsgemäße Polymeroberflächen-Stents, daß alle vorstehend beschriebenen Ausführungsformen und -arten, Herstellungsverfahren und Verwendungen des speziell FK506 enthaltenden Implantats auch für den erfindungsgemäßen Polymeroberflächen-Stent bevorzugt und damit Gegenstände dieser Erfindung sind, solange die Ausführungsformen noch Polymeroberflächen-Stents sind.

Ein weiterer Gegenstand der Erfindung ist auch die Behandlung eines Menschen oder Tieres, der oder das diese Behandlung benötigt, mit oder durch ein erfindungsgemäßes Implantat oder einen Polymeroberflächen-Stent.

im folgenden Abschnitt wird die Erfindung weiter durch Beispiele erläutert, ohne sie darauf zu beschränken.

### Beispiele und Abbildungen:

### Abbildungen:

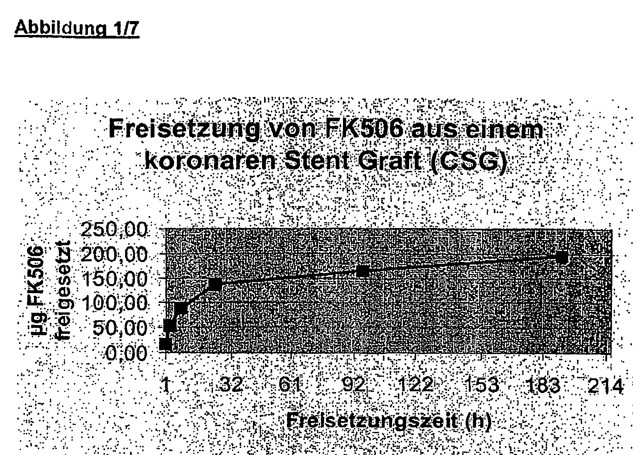

Abbildung 1 zeigt die Freisetzung von FK506 aus einem koronaren Stent Graft, bei dem die aus PTFE bestehende Oberfläche mit FK506 beladen wurde.

Abbildung 2 zeigt die Freisetzung von Candesartan und Quinapril aus einem Stent Graft, bei dem die aus PTFE bestehende Oberfläche mit Candesartan und Quinapril beschichtet wurde.

Abbildung 3 zeigt die Freisetzung von Candesartan und Quinapril aus einem mit Polyurethan beschichteten Stent, bei dem diese Beschichtung mit Candesartan und Quinapril angereichert wurde.

Abbildung 4 zeigt die Freisetzung von Candesartan und Quinapril aus einem mit Polyurethan/Hydrogel-Blend beschichteten Stent, bei dem diese Beschichtung mit Candesartan und Quinapril angereichert wurde.

Abbildung 5 zeigt die Freisetzung von FK506 im Blut nach Implantation entsprechend beschichteter Stents in Kaninchen.

Abbildung 6 zeigt die Fläche der Intima auf implantierten Stents mit und ohne entsprechender Beschichtung mit FK506.

Abbildung 7 zeigt die inflammatorische Reaktion auf Implantation von Stents mit und ohne entsprechender Beschichtung mit FK506.

### Beispiele:

### Beispiel 1:

### Beladung interessanter Verbindungen auf Stent Grafts

Alle Werte sind in µg angegeben.

**Tabelle 1**

| | | | | | |
|---|---|---|---|---|---|
| | | | | | |

| **Wirkstoff / Stent-Art** | **FK 506 (Tacrolimus)** | **Vinblastin** | **Paclitaxel (Taxol)** | **Cisplatin** | **Mitoxantron** |
|---|---|---|---|---|---|
| **A** | 1382 | 74 | 128 | 15* | 116 |
| | 1671 | 125 | 155 | 14* | 142 |
| | 1625 | 238 | 148 | 14* | 113 |
| **Durch-schnitt** | **1559** | **146** | **144** | **14** | **124** |
| **B** | 18 | | | | |
| | 16 | | | | |
| | 21 | | | | |
| **Durch-schnitt** | **18** | | | | |
| **C** | 194 | | | | |
| | 165 | | | | |
| **Durch-schnitt** | **180** | | | | |

| | | | | | |
|---|---|---|---|---|---|
| * gemessen durch AAS A: Versuche mit gelösten Feststoffen, bei denen Stent Grafts mit einer PTFE-Polymerschicht in die Lösung eingetaucht wurden. B: Versuche mit i.v.-Lösungen, bei denen Stent Grafts mit einer PTFE-Polymerschicht in die Lösung eingetaucht wurden. C: Versuche mit i.v.-Lösungen, bei denen mit Polyurethan beschichtete Stents in die Lösung eingetaucht wurden. | | | | | |

### Beispiel 2 :

### Freisetzungsmuster verschieden hergestellter erfindungsgemäßer Stent Grafts wie auch allgemeiner Polymeroberflächen-Stents:

Alle Werte sind in µg angegeben.

Für die Analyse der Wirkstoff-Freisetzung wurde ein Stent bei 37°C in 10 ml PBS-Puffer (stabilisiert mit Na-Azid) at 37°C inkubiert. Nach Ablauf definierter Zeitabschnitte wurden 2 x 1 ml der Lösung entfernt und analysiert. Diese 2 ml wurden durch frischen PBS-Puffer (stabilisiert mit Na-Azid) ersetzt.

In den Tabellen ist der gesamte freigesetzte Gehalt an Wirkstoff in der Lösung angegeben. Das bedeutet, daß die Menge an Wirkstoff in dem für die Analyse entnommenen Puffervolumen zu der am nachfolgenden Zeitpunkt entnommenen Menge hinzuaddiert wurde.

**Tabelle 2:**

| **Vinblastin** | **nach 1 h** | **nach 3 h** | **nach 8 h** | **nach 24 h** | **nach 96 h nach 72 h** |
|---|---|---|---|---|---|
| **Wirkstoff/ Art** | | | | | |
| **A** | 73 und 75 Schnitt: **74** | 108 und 114 Schnitt: **109** | 121 und 126 Schnitt: **124** | 106 und 120 Schnitt: **113** | 132 und 140 (96h) Schnitt: **136** |
| | 37 und 41 Schnitt: **39** | 48 und 51 Schnitt: **50** | 47 und 58 Schnitt: **42** | 57 und 62 Schnitt: **60** | 56 und 57 (72h) Schnitt: **57** |
| | 80 und 86 Schnitt: **83** | 99 und 104 Schnitt: **102** | 108 und 117 Schnitt: **113** | 117 und 127 Schnitt: **122** | 113 und 121 (72h) Schnitt: **117** |

| | | | | | |
|---|---|---|---|---|---|
| A: Versuche mit gelösten Feststoffen, bei denen Stent Grafts mit einer PTFE-Polymerschicht in die Lösung eingetaucht wurden. | | | | | |

**Tabelle 3:**

| **FK 506 (Tacrolimus)** | **nach 1 h** | **nach 3 h** | **nach 8 h** | **nach 24 h** | **nach 96 h** | **nach 192 h** |
|---|---|---|---|---|---|---|
| **Wirkstoff/ Art** | | | | | | |
| **A** | 14 und 13 Schnitt:**14** | 62 und 62 Schnitt: **62** | 92 und 99 Schnitt: **95** | 148 und 145 Schnitt: **147** | 145 und 151 Schnitt: **148** | 195 und 198 Schnitt: **196** |
| **1. Stent** | | | | | | |
| | **nach 1 h** | **nach 3 h** | **nach 8 h** | **nach 24 h** | **nach 96 h** | |
| **A** | 34 und 26 Schnitt:**30** | 56 und 57 Schnitt:**57** | 82 und 78 Schnitt: **80** | 108 und 109 Schnitt: **109** | 159 und 164 **161** | |
| **2. Stent** | | | | | | |
| | | | | | | |
| | **nach 1 h** | **nach 3 h** | **nach 8 h** | **nach 24 h** | **nach 96 h** | |
| **A** | 12 und 9 Schnitt: **11** | 47 und 43 Schnitt: **45** | 101 und 93 Schnitt: **95** | 154 und 155 Schnitt: **151** | 184 und 190 Schnitt: | |
| **3. Stent** | | | | **154** | **187** | |
| | | | | | | |
| | | | | | | |

| | | | | | | |
|---|---|---|---|---|---|---|
| A: Versuche mit gelösten Feststoffen, bei denen Stent Grafts mit einer PTFE-Polymerschicht in die Lösung eingetaucht wurden. | | | | | | |

**Tabelle 4:**

| **FK 506 (Tacrolimus)** | **nach 1 h** | **nach 3 h** | **nach 8 h** | **nach 24 h** | **nach 96 h** | |
|---|---|---|---|---|---|---|
| **Wirkstoff/ Art C** | 19 und 20 Schnitt: **20** | 25 und 26 Schnitt: **26** | 33 und 33 Schnitt: **33** | 41 und 39 Schnitt: **40** | 43 und 38 Schnitt: **41** | |
| **1. Stent** | | | | | | |
| | | | | | | |
| | | | | | | |
| | **nach 1 h** | **nach 3 h** | **nach 8 h** | **nach 24 h** | **nach 96 h** | **nach 264** h |
| **C** | 20 und 27 Schnitt: **24** | 21 und 24 Schnitt: **22** | 26 und 30 Schnitt: **28** | 34 und 31 Schnitt: **32** | 37 und 35 Schnitt: **36** | **85** |
| **2. Stent** | | | | | | |
| | **nach 367 h** | | | | | |
| | 88 und 94 Schnitt: **91** | | | | | |

| | | | | | | |
|---|---|---|---|---|---|---|
| C: Versuche mit i.v.-Lösungen, bei denen mit Polyurethan beschichtete Stents in die Lösung eingetaucht wurden. | | | | | | |

**Tabelle 5:**

| **Paciltaxel** | **nach 1 h** | **nach 3 h** | **nach 8h** | **nach 24 h** | **nach 96 h** | **nach 192 h** |
|---|---|---|---|---|---|---|
| **Wirkstoff/Art** | | | | | | |
| **A** | 0.14 und 0.23 Schnitt: **0.19** | 0.46 und 0.53 Schnitt: **0.50** | 1.42 und 1.25 Schnitt: 1.34 | 1.65 und 1.42 Schnitt: **1.54** | 1.42 und 1.93 Schnitt **1.68** | 2.22 und 2.24 Schnitt: **2.23** |
| | 0.42 und 0.52 Schnitt: **0.47** | 0.90 und 0.90 Schnitt: **0.90** | 1.16 und 1.21 Schnitt: **1.19** | 2.44 und 2.40 Schnitt: **2.42** | 2.79 und 2.78 Schnitt: **2.79** | |
| | 0.20 und 0.16 Schnitt: **0.18** | 0.51 und 0.95 0.89 und 0.94 Schnitt: **0.73** Schnitt: **0.92** | | 2.26 und 2.27 Schnitt: **2.27** | 2.82 und 2.82 Schnitt:**2.82** | |

| | | | | | | |
|---|---|---|---|---|---|---|
| A: Versuche mit gelösten Feststoffen, bei denen Stent Grafts mit einer PTFE-Polymerschicht in die Lösung eingetaucht wurden. | | | | | | |

**Tabelle 6:**

| **Cisplatin** | **nach 1 h** | **nach 3 h** | **nach 8 h** | **Nach 24 h** |
|---|---|---|---|---|
| **Wirkstoff/ Art** | | | | |
| **A** | 11.7 und 11.9 Schnitt: **11.8** | 15.4 und 15.4 Schnitt: **15.4** | 16.3 und 16.5 Schnitt: **16.4** | 16.1 und 15.9 Schnitt: **16.0** |
| | 5.7 und 5.4 Schnitt: **5.5** | 7.8 und 7.7 Schnitt: **7.8** | 9.9 und 9.8 Schnitt: **9.8** | 10.9 und 11.1 Schnitt: **11.0** |
| | 10.0 und 10.0 Schnitt: **10.0** | 11.4 und 11.8 Schnitt: **11.6** | 12.2 und 12.3 Schnitt: **12.2** | 12.4 und 12.2 Schnitt: **12.3** |

| | | | | |
|---|---|---|---|---|
| A: Versuche mit gelösten Feststoffen, bei denen Stent Grafts mit einer PTFE-Polymerschicht in die Lösung eingetaucht wurden. | | | | |

**Tabelle 7:**

| **Mitoxantron** | **nach 1 h** | **nach 3 h** | **nach 8 h** | **nach 24 h** |
|---|---|---|---|---|
| **Wirkstoff/ Art** | | | | |
| **A** | 57 und 86 Schnitt: **71** | 55 und 52 Schnitt: **54** | 49 und 47 Schnitt: **48** | 42 und 49 Schnitt: **45** |
| | 70 und 74 Schnitt: **72** | 80 und 83 Schnitt: **81** | 80 und 82 Schnitt: **81** | 72 und 75 Schnitt: **74** |
| | 29 und 27 Schnitt: **28** | 25 und 27 Schnitt: **26** | 23 und 21 Schnitt: **22** | 24 und 29 Schnitt: **27** |

| | | | | |
|---|---|---|---|---|
| A: Versuche mit gelösten Feststoffen, bei denen Stent Grafts mit einer PTFE-Polymerschicht in die Lösung eingetaucht wurden. | | | | |

### Beispiel 3:

**Herstellungsverfahren (1) für FK506 beschichtete Implantate:**
- 10 mg FK506 werden in 3 ml Ethanol gelöst
- Unbeschichtete Stents aus Edelstahl werden unter Vakuum über Nacht bei Raumtemperatur in die Lösung getaucht.
- Dreimal 1 Minute mit Saline waschen
- Trocknen über Nacht.

### Beispiel 4:

**Herstellungsverfahren (2) für FK506 beschichtete Stent Grafts:**
- Die Dosierung von FK506 kann nach Stentlänge und Durchmesser sowie nach Anwendung im Körper variieren. Hier wurde eine Dosierung von 10 - 200 µg FK506 pro cm Stentlänge verwendet.
- FK506 wird in einem kleinen Glasgefäß in Ethanol gelöst (entsprechend der gewünschten Dosierung) und die Lösung visuell auf Kristalle überprüft.
- Die nicht vorbehandelten Stent Grafts ("JOSTENT Coronary Stent Graft"), bestehend aus einer Sandwich-Konstruktion von zwei Stents aus Edelstahl mit einer PTFE-Membran, werden auf Halterungen montiert.
- Mit einer Pipette wird die entsprechende Menge an FK506-Lösung (5 bis 30 µl) auf den montierten Stent Graft aufpipettiert.
- Dieser Schritt kann nach Belieben wiederholt werden, um größere Mengen FK506 auf das Implantat aufzubringen, im Normalfall ein oder zwei Mal. Im vorliegenden Fall wurde der Vorgang einmal wiederholt.
- Nachdem der Stent Graft vollständig beladen ist, wird er vorsichtig von der Halterung geschoben.
- Nach kurzem Trocknen an der Luft (ca. 5 min), gegebenenfalls unter Wärmezufuhr, können die Stents verpackt werden.
- Alle Stents werden unter der Lupe auf Medikamentenausflockungen untersucht und gegebenenfalls verworfen.

### Beispiel 5:

**Herstellungsverfahren (3) für FK506 beschichtete Stent, die einen keramischen Überzug besitzen:**
- 50 mg FK506 werden in einem Glasgefäß in 10 ml Ethanol gelöst. Von dieser Stammlösung werden bei Bedarf alle weiteren Konzentrationen durch Verdünnung hergestellt (Verdünnungen zwischen 1:1 und 1:20).
- Die Lösung wird visuell auf Kristalle überprüft.
- Die unbeladenen, mit einer Aluminiumoxid-Schicht (gemäß PCT-Anmeldung WO 00/25841, siehe auch Beispiel 7 zu keramischer Beschichtung) überzogenen Stents werden auf Halterungen montiert.
- Mit einer Injektionsspritze wird die FK506-Lösung (5 bis 50 µl) tropfenweise auf den Stent aufgetragen. Dabei sollte die Lösung über den kompletten Stent verteilt sein.
- Der beladene Stent wird vorsichtig von der Halterung genommen.
- Nach kurzem Trocknen an der Luft (ca. 5 min), gegebenenfalls unter Wärmezufuhr, können die Stents verpackt werden.
- Alle Stents werden unter der Lupe auf Medikamentenausflockungen untersucht und gegebenenfalls verworfen.

### Beispiel 6:

**Neues alternatives Herstellungsverfahren (4) (u.a. für FK506 aber auch für andere Wirkstoffe anwendbar), insbesondere zur Herstellung steriler Stents, Stent Grafts oder bzw. Polymeroberflächen-Stents:**
- Es werden kleine Injektionsflaschen verwendet, die nicht sehr viel größer als der Stent sind.
- Sterile koronare Stent Grafts (CSG's) werden steril in die sterilen Injektionsflaschen gegeben.
- 0,5 ml steril-filtrierter FK506-Lösung (3.3 mg/ml in Ethanol) werden in die Flasche hinzugegeben.
- Die Flachen werden mit Gummi-Stopfen verschlossen.
- Eine sterile Injektions-Kanüle mit sterilem Filter wird durch die Mitte des Gummi-Stopfens gestochen.
- Die Flaschen werden in einem Exsikator unter Vakuum horizontal auf einem Rollengerät aufgestellt.
- Die Flaschen werden über Nacht unter Vakuum gerollt.
- Die Injektions-Nadel wird entfernt.
- Es wird nicht gespült.
- Die sterilen CSG's sind einsatzbereit.

### Beispiel 7:

**Eine Auswahl möglicher Wirkstoffe für einen wirkstoff-freisetzenden Stent, insbesondere mit mehreren Schichten, beispielsweise Polymeroberflächen-Stents bzw. Stent Grafts etc.**

Die Beladungsmethoden werden als technische Ansätze unten beschrieben.

Die aufgezählten Wirkstoffe umfassen auch Derivate sowie alle Arten von Salzen, Enantiomeren, Razematen, Basen oder freien Säuren.

Besonders interessant sind hier Stents, Stent Grafts bzw. Polymeroberflächen-Stents, die mindestens einen, zwei oder drei der unten aufgezählten Wirkstoffe enthalten und entsprechend freisetzen.

Dabei werden die aufgezählten Wirkstoffe nach ihrem bevorzugten Freisetzungsprofil bzw. dem Freisetzungszeitraum in die Gruppen 1 - 3 eingeteilt.

Bevorzugt ist es dabei auch, wenn die Stents, Stent Grafts bzw. Polymeroberflächen-Stents Wirkstoffe aus verschiedenen Gruppen enthalten.

**Tabelle 8**

| **Phase I- Vasodilation (Gruppe 1)** | | |
|---|---|---|
| **Wirkstoffe, die insbesonders innerhalb der ersten 24-72 h nach Setzen des Stents freigesetzt werden.** | | |
| **Wirkstoff** | | |
| Molsidomin, Linsidomin, Natrium-Nitroprussid, Nitroglycerin, NO-donoren allgemeinl | | |
| Stimulatoren der löslichen Guanylat- Cyklase wie BAY 41-2272 (5-(Cyclopropyl-2-[1-(2-fluoro-benzyl)-1 H-pyrazolo[3,4-n]pyridin-3-yl]-pyrimidin-4-ylamin) | | |
| Hydralazin | | |
| Verapamil, Diltiazern, Nifedipin, Nimodipine und andere Ca2⁺-Kanal- Blocker | | |
| Captopril, Enalapril, Lisinopril, Quinapril und andere Inhibitoren der angiotensin-umwandelnden Enzyme | | |
| Losartan, Candesartan, Irbesartan, Valsartan und andere Angiotensin 11 -Rezeptor Antagonisten | | |

**Tabelle 9**

| **Phase II - Inhibition von Entzündung, Immunsuppression, Promotion des Zellwachstums endothelialer Zellen, Inhibition der Zell-Migration (Gruppe 2)** | | |
|---|---|---|
| **Wirkstoffe, die insbesonders innerhalb der ersten 2-21 Tage nach Setzen des Stents freigesetzt werden.** | | |
| **Wirkstoff** | | |
| Dexamethason, Betamethason, Prednison und andere Corticosteriode | | |
| 17-beta-Östradiol | | |
| FK506 (Tacrolimus) | | |
| Cyclosporin | | |
| Mycophenolsäure | | |
| VEGF, VEGF-Rezeptor Aktivatoren | | |
| Tranilast | | |
| Meloxicam, Celebrex, Vioxx und andere COX-2 Antagonisten | | |
| Indomethacin, Diclofenac, Ibuprofen, Naproxen und andere COX-1 Inhibitoren | | |
| Plasminogen-Aktivator-Inhibitor-1 und andere Serpine | | |
| Thrombin inhibitoren wie Hirudin, Hirulog, Agratroban, PPACK etc. | | |
| Interleukin-10 | | |

**Tabelle 10**

| **Phase III - Inhibition der Zell-Proliferation (Gruppe 3)** | | |
|---|---|---|
| **Wirkstoffe, die insbesonders innerhalb der ersten 14 Tage bis 3 Monate nach Setzen des Stents freigesetzt werden.** | | |
| **Wirkstoff** | | |
| Sirolimus, SDZ RAD (40-O-(2-hydroxyethyl)-Rapamycin und andere Rapamycin-Derivate | | |
| PDGF-Antagonisten | | |
| Paclitaxel | | |
| Cisplatin | | |
| Vinblastin | | |
| Mitoxantrone | | |
| Combretastatin A4 | | |
| Topotecan | | |
| Methotrexat | | |
| Flavopiridol | | |

Eine lokale Anwendung des Wirkstoffs wird durch direkte Abgabe von der wirkstoffbeladenen Oberfläche eines koronaren oder peripheren Stents erzielt. Eine wirkstoffbeladene Oberfläche eines Stents kann durch Verwendung verschiedener technologischer Ansätze erreicht werden. Jeder dieser Ansätze kann so durchgeführt werden, daß der Wirkstoff von der Oberfläche entweder in einem kurzen (Stunden) oder einem ausgedehnten Zeitraum (Tage) freigesetzt wird. Die Freisetzungskinetik kann dadurch angepaßt werden, daß spezifische Modifikationen an der Oberfläche vorgenommen werden, z.B. hydrophobe oder hydrophile Seitenketten eines polymeren Trägers oder eine keramische Oberfläche.

### • Keramische Beschichtung

Eine Aluminiumoxid-Beschichtung (Patentanmeldungen DE 19855421, DE 19910188, WO 00/25841) mit poröser Oberfläche kann mit Wirkstoff (beispielsweise FK506 mit Mengen zwischen 10 µg und 10 mg) entweder durch Eintauchen, Aufsprühen oder eine vergleichbare Technik beladen werden. Die Dosis des Wirkstoffes hängt von der Art des Zielgefäßes und dem Zustand des Patienten ab und wird so gewählt, daß Proliferation, Migration und T-Zell-Antwort ausreichend inhibiert werden, ohne daß der Heilungsprozeß behindert wird. Der Wirkstoff kann als wässrige oder organische Lösung, beispielsweise in DMSO, DMF und Ethanol, verwendet werden. Nach dem Sprühen oder Eintauchen (unter schwachem Vakuum) wird der behandelte Stent getrocknet und der Vorgang 1 bis 5-mal wiederholt. Nach dem letzten Trockenschritt wird der Stent 1 min lang bei Raumtemperatur in Wasser oder isotonischer Saline gespült und anschließend wieder getrocknet. Der Wirkstoffgehalt kann nach Herauslösen des Wirkstoffs mit einem geeigneten Lösungsmittel durch Standardmethoden (HPLC, LC-MS) analysiert werden. Freisetzungskinetiken können unter Verwendung einer Standard-Freisetzungsmeßapparatur gemessen werden.

### • PTFE Membran: Stent Graft

Hier wird ein zu oben beschrieben identischer Ansatz verwendet. Der Wirkstoff wird in den Vertiefungen der porösen PTFE-Membran eingelagert.

### • Allgemeine polymerische Beschichtung

Verschiedene Polymere sind für eine Wirkstoffbeladung geeignet: Methacrylate-Polymere, Polyurethan-Beschichtungen, PTFE-Beschichtungen oder Hydrogel-Beschichtungen. Der Wirkstoff kann entweder auf die End-Oberfläche aufgetragen werden (s.o.) oder wird direkt der Polymerisationslösung beigegeben. In den übrigen Details entspricht dieser technische Ansatz den oben bereits beschriebenen.

### • Mechanischer Ansatz

Der mechanische Ansatz beruht auf Vertiefungen, die in die Stent-Streben mittels eines Schneid-Lasers eingebracht werden. Diese Vertiefungen können dann mit Wirkstoff gefüllt werden. Der mechanische (Vertiefungen)-Ansatz kann mit einer dünnen bioabbaubaren Beschichtung kombiniert werden, die selbst wirkstoffbeladen ist. Nach einer anfänglichen Freisetzung aus der bioabaubaren Beschichtung kann aus den wirkstoffgefüllten Vertiefungen langfristig Wirkstoff freigesetzt werden. In den übrigen Details entspricht dieser technische Ansatz den oben bereits beschriebenen.

### Beispiel 8:

### Wirkstofffreisetzung von Candesartan und Quinapril von (Polymer-) beschichteten Implantaten:

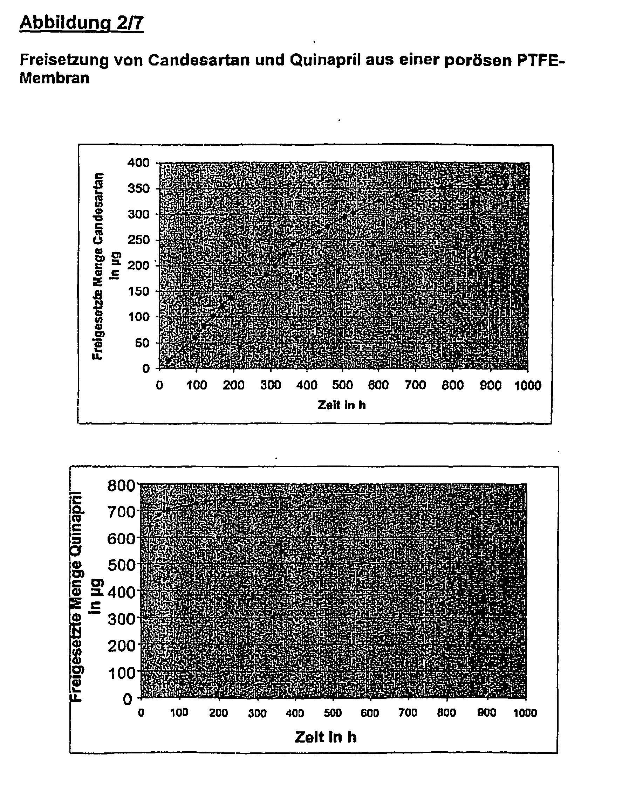

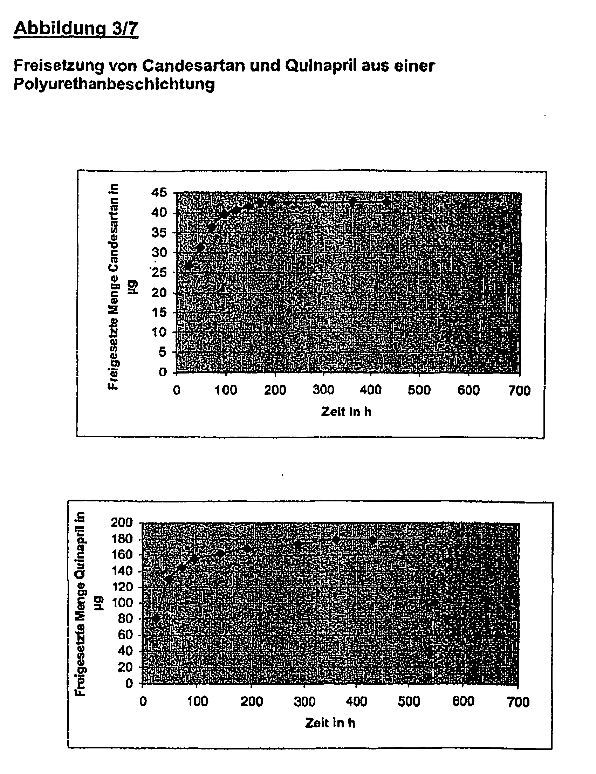

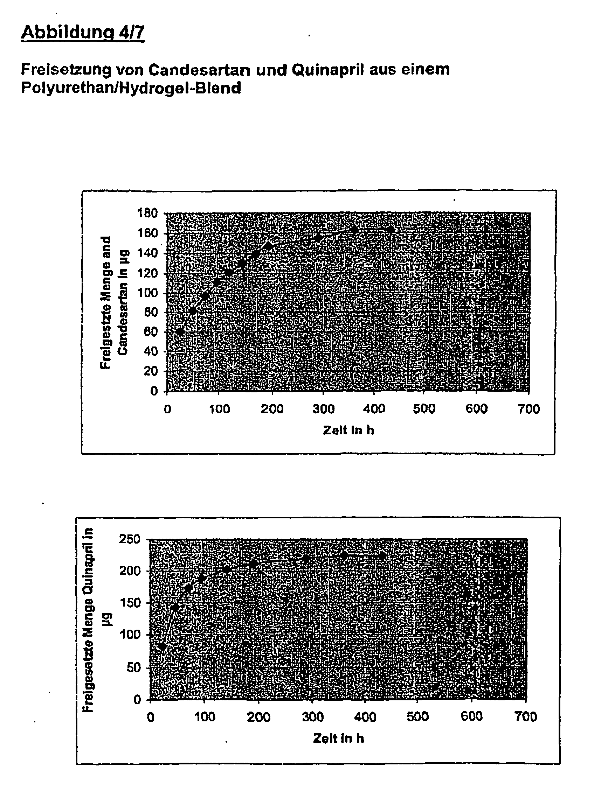

a) Stents wurden mit einer porösen PTFE-Membran ausgestattet. Diese Membran wurde anschließend mit einem Wirkstoffgemisch aus Candesartan und Quinapril beladen (je 1 mg). Die beiden Wirkstoffe werden gleichzeitig freigesetzt. Die Freisetzung wurde in PBS (phosphate buffered saline) ermittelt. Abbildung 2 zeigt die Wirkstofffreisetzung von Candesartan und Quinapril vom Stent.
b) Offenzellige Edelstahlstents wurden mit einer Polyurethanbeschichtung versehen. Ein Wirkstoffgemisch aus Candesartan und Quinapril (je 15% bezogen auf den Polyurethangehalt) wurde in eine 5%ige Lösung des Polyurethans in Dimethylacetamid eingebracht und mittels eines Sprühverfahrens auf den Stent aufgebracht. Abbildung 3 zeigt die entsprechende Wirkstofffreisetzung.
d) Offenzellige Edelstahlstents wurden mit einer Polyurethan/Hydrogelbeschichtung versehen. Ein Wirkstoffgemisch aus Candesartan und Quinapril (je 15% bezogen auf den Polymergehalt) wurde in eine 5%ige Lösung des Polymerblends in Dimethylacetamid eingebracht und mittels eines Sprühverfahrens auf den Stent aufgebracht. Abbildung 4 zeigt die entsprechende Wirkstofffreisetzung.

### Beispiel 9:

### Studie zur Freisetzungkinetik und Wirkungsweise von FK506 im Tier

Koronare Edelstahlstents (JOSTENT Flex, 16 mm) wurden mit einer keramischen Schicht aus Aluminiumoxid überzogen. Diese Beschichtung diente als Träger von FK506 (wie in Beispiel 5 sowie Beispiel 7 unter "Keramische Beschichtung" beschrieben). In diesem Fall wurden die Stents mit insgesamt 60 µg FK506 beladen. In einer Tierstudie wurden diese FK506 beschichteten Stents sowie normale Stents ohne Beschichtung in Kaninchen (n=7) in die Karotis-Arterie neuseeländischer Kaninchen implantiert. Untersucht wurde die Freisetzung von FK506 sowie die Wirkung von FK506 auf das Wachstum der Intima und die Bildung von Makrophagen und Lymphozyten.

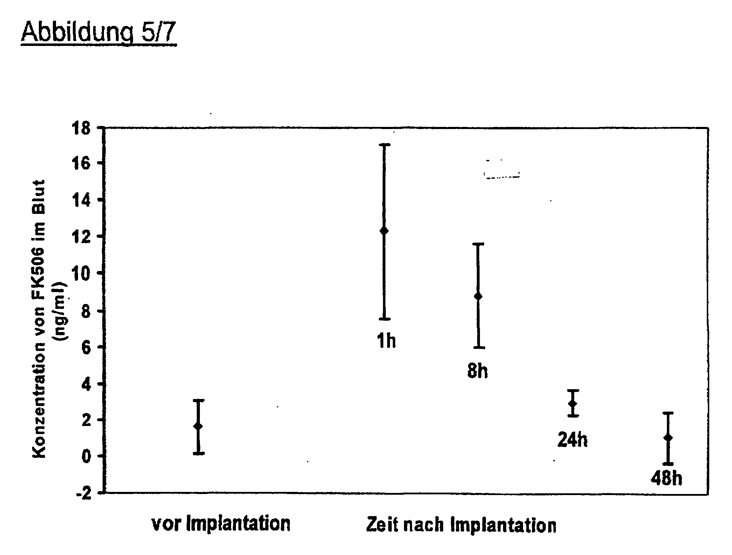

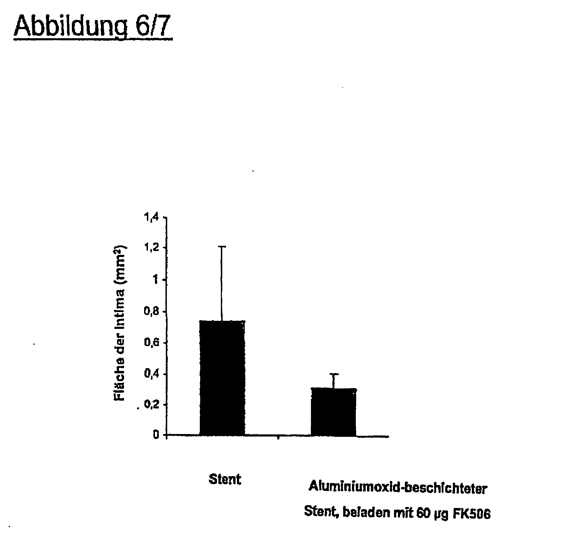

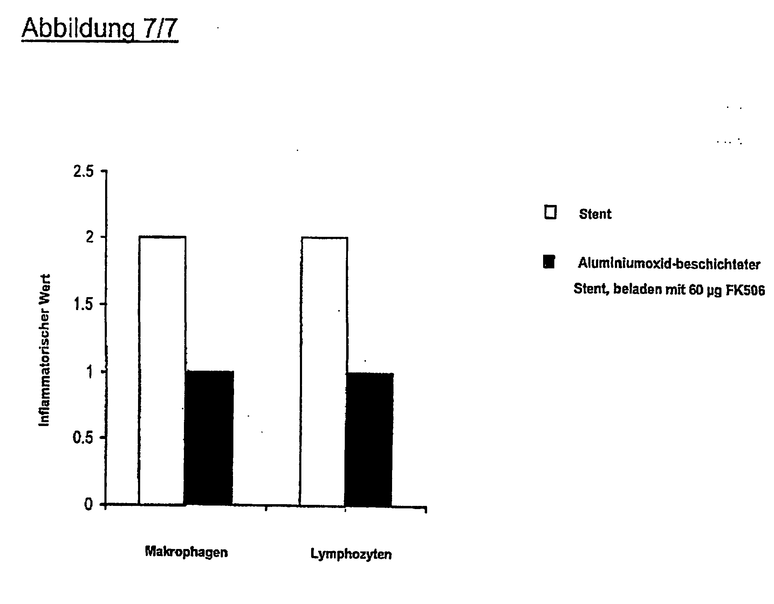

Bestimmt wurden die Freisetzung von FK506 durch HPLC-Bestimmung der Menge an FK506 im Blut der Kaninchen nach 1 h, 8 h, 24 h und 48 h. In Abbildung 5 sieht man deutlich eine sehr gute Freisetzungskinetik von FK506 über die Zeit. Die Kaninchen wurden nach 28 Tagen getötet und die implantierten Stents untersucht. Dabei wurde die Fläche der neugewachsenen Intima (Neointima) innerhalb des Stents unter dem Lichtmikroskop quantifiziert. Abbildung 6 kann man eindeutig entnehmen, daß die Bildung von Neointima bei den keramisch beschichteten Stents, die mit FK506 beladen wurden, deutlich reduziert ist im Vergleich zu normalen Stents. Bei einer Beladung mit 60 µg FK506 findet man eine Reduktion von 53%. Begleitet wurde diese Reduktion zudem durch eine spürbare Verringerung der Entzündungsherde. Wie man in Abbildung 7 erkennen kann, ist im Vergleich zum normalen Stent die Makrophagensowie die Lymphozyten-Bildung deutlich reduziert.

Der Einsatz von Stents mit einer keramischen Beschichtung, auf die FK506 aufgebracht wird, führt somit nach der Implantation zu deutlicher Reduktion von Neointima sowie von Entzündungsherden.

## Patentansprüche

1. Intrakavernöses, vorzugsweise intravaskuläres Implantat enthaltend in chemisch kovalent oder nicht-kovalent gebundener oder physikalisch fixierter Form FK506 sowie gegebenenfalls mindestens einen weiteren Wirkstoff, wobei das Implantat ein Substrat aufweist, das mit FK506 beschichtet ist, und das Substrat wenigstens eine aus einem Metall oder einer Metalllegierung bestehende, homogene oder aus verschiedenen Strängen gebildete, geschlossene oder durchbrochene Schicht oder Oberfläche aufweist.

2. Implantat gemäß Ansprüch 1, **dadurch gekennzeichnet, daß** das Implantat zur Behandlung oder Prophylaxe koronaren oder peripheren Gefäßverengungen oder verschlüssen, insbesondere von Verengungen bzw. Stenosen oder Restenosen, vorzugsweise zur Prophylaxe von Restenose geeignet ist.

3. Implantat gemäß einem der Ansprüche 1 oder 2, **dadurch gekennzeichnet, daß** das Implantat wenigstens eine aus einem Polymer bestehende, homogene oder aus verschiedenen Strängen gebildete, geschlossene oder durchbrochene Schicht oder Oberfläche aufweist.

4. Implantat gemäß Anspruch 3, **dadurch gekennzeichnet, daß** wenigstens eine Polymerschicht ganz oder teilweise eine aus einem Metall oder einer Metalllegierung bestehende, homogene oder aus verschiedenen Strängen gebildete, geschlossene oder durchbrochene Schicht oder Oberfläche, vorzugsweise eine aus einem Metall oder einer Metalllegierung bestehende, gegebenenfalls gitterförmige Struktur bedeckt.

5. Implantat gemäß Anspruch 3, **dadurch gekennzeichnet, daß** das Implantat wenigstens eine aus einem. Metall oder einer Metalllegierung bestehende, homogene oder aus verschiedenen Strängen gebildete, geschlossene oder durchbrochene Schicht oder Oberfläche und wenigstens eine aus einem Polymer bestehende, homogene oder aus verschiedenen Strängen gebildete, geschlossene oder durchbrochene Schicht oder Oberfläche aufweist.

6. Implantat gemäß Anspruch 5, **dadurch gekennzeichnet, daß** die aus einem Metall oder einer Metalllegierung bestehende Schicht oder Oberfläche eine aus einem Metall oder einer Metalliegierung bestehende, gegebenenfalls gitterförmige, Struktur ist und/oder die aus einem Polymer bestehende Schicht oder Oberfläche homogen geschlossen oder gewebt ist und/oder wasser- und/oder korpuskelundurchlässig ist und/oder die Abfolge der Schichten und Oberflächen von Außen nach Innen Metall-Polymer, Polymer-Metall, Metall-Polymer-Metall oder Polymer-Metall-Polymer ist und/oder entweder die aus einem Polymer bestehende Schicht oder Oberfläche nicht chemisch (kovalent oder nichtkovalent) mit der aus einem Metall oder einer Metalllegierung bestehenden Schicht oder Oberfläche verbunden ist oder die aus einem Polymer bestehende Schicht oder Oberfläche mittels eines Klebstoffes mit der aus einem Metall oder einer Metalllegierung bestehenden Schicht oder Oberfläche verbunden ist.

7. Implantat gemäß einem der Ansprüche 3 bis 6, **dadurch gekennzeichnet, daß** das Polymer ausgewählt ist aus Dacron; Polytetrafluorethylen (PTFE/Teflon), ausdehnbar oder nicht ausdehnbar; Polyurethan; Methacrylat-Polymeren; Hydregel oder Hydrogel/Polyurethan-Blend, vorzugsweise aus Polytetrafluorethylen (PTFE), ausdehnbar oder nicht ausdehnbar; oder Polyurethan; insbesondere aus PTFE.

8. Implantat gemäß einem der Ansprüche 1 bis 7, **dadurch gekennzeichnet, daß** das Implantat ein Steht ein Stent Graft, ein Graft, ein Graft-Verbinder, ein Führungsdraht, ein Katheter oder eine Katheterpumpe, vorzugsweise ein Stent, ein Stent Graft, ein Graft oder ein Graft-Verbinder, insbesondere ein Stent oder ein Stent Graft ist.

9. Implantat gemäß einem der Ansprüche 1 bis 8 ,**dadurch gekennzeichnet, daß** das Substrat einen keramischen Überzug, insbesondere aus Aluminiumoxid oder Iridiumoxid, aufweist, an den FK506 gebunden ist.

10. Implantat gemäß Anspruch 9, **dadurch gekennzeichnet daß** der keramische Überzug ganz oder teilweise mit einem polymeren, vorzugsweise biologisch abbaubaren, Überzug bedeckt ist, an den gegebenfalls FK506 und/oder ein weiterer Wirkstoff gebunden ist oder in dem FK506 und/oder ein weiterer Wirkstoff vor Aufbringung des Überzugs gelöst wurde.

11. Implantat gemäß einem der Ansprüche 1 bis 10, **dadurch gekennzeichnet, daß** das Substrat einen polymeren Überzug, insbesondere aus Methacrylat-Polymeren, Polyurethan, PTFE, Hydrogel oder Hydrogel/Polyurethan-Blend, insbesondere PTFE, aufweist, an den FK506 gebunden ist oder in dem FK506 vor Aufbringung des Überzugs gelöst wurde.

12. Implantat gemäß einem der Ansprüche 1 bis 11, **dadurch gekennzeichnet, daß** das Metall des Implantats mittels Laser eingebrachte Vertiefungen aufweist, die mit FK506 gefüllt sind.

13. Implantat gemäß Anspruch 12, **dadurch gekennzeichnet, daß** das mit FK506-gefüllten Vertiefungen versehene Metall oder mindestens die Vertiefungen mit einem biologisch abbaubaren Polymermaterial überzogen wird/werden, wobei gegebenenfalls FK506 an den polymeren Überzug gebunden wird oder FK506 vor Aufbringung des Überzugs in dem Polymermaterial gelöst wurde.

14. Implantat gemäß einem der Ansprüche 1 bis 13, **dadurch gekennzeichnet, daß** FK506 in Form von beladenen Nanopartikeln oder Liposomen vorliegt.

15. Implantat gemäß einem der Ansprüche 1 bis 14 herstellbar durch ein Verfahren, bei dem
a) ein wenigstens eine aus einem Metall oder einer Metall-Legierung bestehende, homogene oder aus verschiedenen Strängen gebildete, geschlossene oder durchbrochene Schicht oder Oberfläche aufweisendes Substrat, das mit einem keramischen Überzug, insbesondere aus Aluminiumoxid oder Iridiumoxid, überzogen wird, eingesetzt wird
oder
b) ein wenigstens eine aus einem Polymer bestehende, homogene oder aus verschiedenen Strängen gebildete, geschlossene oder durchbrochene Schicht oder Oberfläche aufweisendes Substrat eingesetzt wird,
oder
c) ein Substrat das mit einem polymerisierten oder auf der Oberfläche polymerisierenden Überzug, insbesondere aus Methacrylat-Polymeren, Polyurethan, PTFE, Hydrogel oder Hydrogel/Polyurethan-Blend, überzogen wird, eingesetzt wird
oder
d) ein Substrat mit wenigstens einer aus einem Metall oder einer Metall-Legierung bestehenden, homogenen oder aus verschiedenen Strängen gebildeten, geschlossenen oder durchbrochenen Schicht oder Oberfläche, in die mittels eines Lasers Vertiefungen eingebracht werden, die mit FK506 gefüllt werden, und dann das Substrat mit einem polymerisierten oder auf der Oberfläche polymerisierenden bioabbaubaren Überzug überzogen wird, eingesetzt wird,
e) dann das Substrat gemäß a), b), c) oder d) mit einer FK506-Lösung in wäßrigem oder organischem Lösungsmittel in Kontakt gebracht wird, beispielsweise durch Beträufeln, Besprünen oder Eintauchen gegebenenfalls unter Vakuum,
f) dann gegebenenfalls das so behandelte Substrat getrocknet wird, vorzugsweise bis zur Entfernung des Lösungsmittels aus Schritt e),
g) dann gegebenenfalls Schritt e) gegebenenfalls gefolgt von Schritt f) wiederholt wird, vorzugsweise mehrfach, insbesondere 1 bis 5-fach, sowie
h) gegebenenfalls anschließend das behandelte Substrat ein- oder mehrfach mit Wasser oder isotoner Saline gespült wird und
i) gegebenenfalls anschließend getrocknet wird.

16. Implantat gemäß Anspruch 15, **dadurch gekennzeichnet, daß** in Schritt e) FK506 in Alkohol gelöst wird, vorzugsweise in Ethanol, insbesondere in einer Konzentration von 0,5 - 5 g/l FK506 in Ethanol und/oder daß in Schritt e) das Implaritat unter Vakuum vorzugsweise Ober Nacht durch Eintauchen mit einer FK506-Lösung in wäßrigem oder organischem Lösungsmittel in Kontakt gebracht wird und/oder die Schritte f) und/oder g) nicht ausgeführt werden und/oder bei Schritt h) das Implantat mehrfach mit Saline gewaschen wird und/oder in Schritt i) das Implantat über Nacht getrocknet wird.

17. Implantat gemäß Anspruch 15, **dadurch gekennzeichnet, daß** in Schritt e) das Implantat, vorzugsweise steril, in ein vorzugsweise steriles Gefäß mit einem sich nach Ende einer Perforation verschließenden, perforierbarem Verschluß, beispielsweise in eine injektions-Flasche, eingebracht wird, FK506-Losung. Vorzugsweise steril, in das Gefäß gefüllt wird, dieses mit dem sich nach Ende einer Perforation verschließenden, perforierbarem Verschluß verschlossen wird, ein dünnes, vorzugsweise steril, luftdurchlässiges. Belüftungsrohr, beispielsweise eine Kanüle; perforierend durch den Verschluß gesteckt wird, ein Vakuum angelegt und vorzugsweise die, FK506-Lösung bewegt wird und abschließend, vorzugsweise nach Ablauf von ca. 12 h, das dünne, vorzugsweise steril, luftdurchlässige Belüftungsrohr entfernt wird und/oder daß in Schritt e) FK506 in Alkohol gelöst wird, vorzugsweise in Ethanol, insbesondere in einer Konzentration von 3,3 mg FK506 in 1 ml Ethanol und/oder daß das Implantat bis zum Einsatz im vorzugsweise sterilen verschlossenen Glasgefäß von Schritt e) verbleibt und/oder daß die Schritte f) bis i) unterbleiben.

18. Implantat gemäß einem der Ansprüche 3 bis 8 herstellbar durch ein Verfahren, bei dem vor Bildung wenigstens einer aus einem Polymer bestehenden, geschlossenen oder durchbrochenen Schicht oder Oberfläche oder eines polymeren Überzugs des Implantats FK506 im Polymerisations-Material gelöst wurde.

19. Implantat gemäß einem der Ansprüche 1 bis 18, **dadurch gekennzeichnet, daß** nach Implantierung des Implantats FK506 freigesetzt wird.

20. Implantat gemäß Anspruch 19, **dadurch gekennzeichnet, daß** die Freisetzung verzögert erfolgt.

21. Implantat gemäß Anspruch 20, **dadurch gekennzeichnet, daß** FK506 vom Implantat über einen Zeitraum von 24 h, vorzugsweise 48 h, insbesondere mehr als 96 h nach Implantierung freigesetzt wird.

22. Implantat gemäß Anspruch 20, **dadurch gekennzeichnet, daß** das FK506 vom Implantat nach Implantation
a) innerhalb von < 48 h oder
b) Ober mindestens 48 h, vorzugsweise Ober mindestens 7 Tage, insbesondere über mindestens 2 bis zu 21 Tagen freigesetzt wird, oder daß
c) das Implantat beide Freisetzungsmuster a) und b) zeigt.

23. Implantat gemäß einem der Ansprüche 1 bis 22, **dadurch gekennzeichnet, daß** das Implantat mindestens einen weiteren Wirkstoff enthält, vorzugsweise einen pharmazeutischen Wirkstoff, insbesondere einen weiteren Wirkstoff ausgewählt aus den folgenden Wirkstoffen bzw. deren Derivaten -
(Gruppe 1:) Molsidomin, Linsidomin, Natrium-Nitroprussid, Nitroglycerin oder allgemeinen NO-Donoren ; Stimulatoren der löslichen Guanylat-Cyclase (sGC/soluble guanylate cyclase). beispielsweise BAY 41-2272 (5-(Cyclopropyl-2-[1-(2-fluoro-benzyl)-1 H-pyrazolo[3,4-n]pyridin-3-yl]-pyrimidin-4-ylamine) ; Hydralazin; Verapamil, Diltiazem, Nifedipin, Nimodipin oder anderen Ca²⁺-Kanal-Blockern ; Captopril, Enalapril, Lisinopril, Quinapril oder anderen Inhibitoren Angiotensin umwandelnder Enzyme (anglotensin converting enzyme inhibitors); Losartan, Candesartan, Irbesartan, Valsartan oder anderen Antagonisten des Angiotensin 11-Rezeptors;
(Gruppe 2:) Dexamethason, Betamethason, Prednison oder anderen Corticosterioden; 17 -beta-Östradiol; Cyclosporin; Mycophenol-Säure; VEGF, VEGF-Rezeptor Aktivatoren; Tranilast; Meloxicam, Celebrex, Vioxx oder anderen COX-2 Antagonisten; Indomethacin, Diclofenac, Ibuprofen, Naproxen oder anderen COX-1-Inhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK; Interleukin-10;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavopiridol;
Actinomycin D; Rheopro/Abciximab oder Probucol.

24. Implantat gemäß Anspruch 23, **dadurch gekennzeichnet, daß**, wenn der weitere Wirkstoff ausgewählt ist aus Gruppe 1, dieser vom Implantat innerhalb der ersten 24 - 72 h nach Implantation freigesetzt wird und/oder, wenn der weitere Wirkstoff ausgewählt ist aus Gruppe 2, dieser vom Implantat innerhalb der ersten 48 h - 21 Tagen nach Implantation freigesetzt wird und/oder, wenn der weitere Wirkstoff, ausgewählt ist aus Gruppe 3, dieser vom Implantat innerhalb von 14 Tagen bis 3 Monaten nach Implantation freigesetzt wird.

25. \/erfahren zur Herstellung eines Implantats gemäß einem der Ansprüche 1 bis 22 mit folgenden Schritten.
a) ein wenigstens eine aus einem Metall oder einer Metall-L.egierung bestehende, homogene oder aus verschiedenen Strängen gebildete, geschlossene oder durchbrochene Schicht oder Oberfläche aufweisendes Implantat gemäß einem der Ansprüche 1, 2, oder 4 bis 8, das mit einem keramischen Überzug, insbesondere aus Aluminiumoxid oder Iridiumoxid, überzogen wird,
oder
b) ein wenigstens eine aus einem Polymer bestehende, homogene oder aus verschiedenen Strängen gebildete, geschlossene oder durchbrochene Schicht oder Oberfläche aufweisendes Implantat gemäß einem der Ansprüche 3 bis 8,
oder
c) ein Implantat gemäß einem der Ansprüche 1 bis 8, das mit einem polymerisierten oder auf der Oberfläche polymemierenden Überzug, insbesondere aus Methacrylat-Polymeren, Polyurethan, PTFE, Hydrogel oder Hydrogel /Polyurethan-Blend, überzogen wird,
oder
d) ein implantat gemäß einem der Ansprüche 1,2 oder 4 bis 8 mit wenigstens einer aus einem Metall oder einer Metall-egierung bestehenden, homogenen oder aus verschiedenen Strängen gebildeten, geschlossenen oder durchbrochenen Schicht oder Oberfläche, in die mittels eines Lasers Vertiefungen eingebracht werden, die mit FK506 gefüllt werden, und dann das Implantat mit einem polymerisierten oder auf der Oberflache polymerisierenden vorzugsweise bioabbaubaren Überzug überzegen wird, wird eingesetzt,
e) dann wird das Implantat gemäß a), b), c) oder d) mit eine FK506-Lösung in wäßrigem oder organischem Lösungsmittel in Kontakt gebracht, beispielsweise durch Beträufeln, Sprühen oder Eintauchen, gegebenenfalls unter Vakuum,
f) dann wird gegebenenfalls das Implantat getrocknet, vorzugsweise bis das Lösungsmittel aus Schritt e) verdampft ist,
g) dann wird gegebenenfalls Schritt e) gegebenenfalls gefolgt von Schritt f), vorzugsweise mehrfach, insbesondere 1 bis 5-fach, wiederholt sowie
h) gegebenenfalls wird anschließend das Implantat ein- oder mehrfach mit Wasser oder isotoner Saline gespült und
i) gegebenenfalls wird anschließend getrocknet.

26. Verfahren gemäß Anspruch 25, **dadurch gekennzeichnet, daß** in Schritt e) FK506 in Alkohol gelöst wird, vorzugsweise in Ethanol, insbesondere in einer Konzentration von 0,5 - 5 g/l FK506 in Ethanol und/oder daß in Schritt e) das Implantat unter Vakuum vorzugsweise Ober Nacht durch Eintauchen mit eine FK506-Lösung in wäßrigem oder organischem Lösungsmittel in Kontakt gebracht wird und/oder daß die Schritte f) und/oder g) nicht ausgeführt werden und/oder daß bei Schritt h) das Implantat mehrfach mit Saline gewaschen wird und/oder daß in Schritt i) das Implantat über Nacht getrocknet wird.

27. Verfahren gemäß Anspruch 25, **dadurch gekennzeichnet, daß** in Schritt e) das Implantat, vorzugsweise steril, in ein vorzugsweise steriles Gefäß mit einem sich nach Ende einer Perforation verschließenden, perforierbarem Verschluß, beispielsweise in eine Injektions-Flasche, eingebracht wird, FK506-Lösung, vorzugsweise steril, in das Gefäß gefüllt wird, dieses mit dem sich nach Ende einer Perforation verschließenden, perforierbarem Verschluß verschlossen wird ein dünnes, vorzugsweise steril, luftdurchlässiges Belüftungsrohr, beispielsweise eine Kanüle, perforierend durch den Verschluß gesteckt wird, ein Vakuum angelegt und vorzugsweise die" FK506-Lösung in Bewegung gehalten wird und abschließend vorzugsweise nach Ablauf von ca. 12h das dünne, vorzugsweise steril, luftdurchlässige Belüftungsrohr entfernt wird und/oder daß in Schritt e) FK506 in Alkohol gelöst wird, vorzugsweise in Ethanol, insbesondere in einer Konzentration von 3,3 mg FK506 in 1 ml Ethanol und/oder daß das Implantat bis zum Einsatz im sterilen verschlossenen Glasgefäß von Schritt e) verbleibt und/oder daß die Schritte f) bis i) unterbleiben.

28. Verfahren zur Herstellung von implantaten gemäß einem der Ansprüche 3 bis 8, bei dem vor Bildung wenigstens einer aus einem Polymer bestehenden, geschlossenen oder durchbrochenen Schicht oder Oberfläche oder eines polymeren Überzugs des Implantats FK506 im Polymerisations-Material gelöst wurde.

29. Verfahren zur Herstellung von mit zumindest FK 506 enthaltenden Wirkstoffen beschichteter, intrakavernöser, vorzugeweise intravaskulärer Implantate mit folgenden Schritten:
a) das Implantat wird, vorzugsweise steril, in ein vorzugsweise steriles Gefäß mit einem sich nach Ende einer Perforation verschließenden, perforierbarem Verschluß, beispielsweise in eine lnjektions-Flasche eingebracht
b) vorzugsweise sterile Wirkstofflösung, vorzugsweise in einem organischen Lösungsmittel mit geringem Dampfdruck, insbesondere in Alkohol wie Ethanol oder Methanol, wird in das Gefäß eingebracht.
c) das Gefäß wird mit dem sich nach Ende einer Perforation verschließenden, perforierbarem Verschluß verschlossen,
d) ein dünnes, vorzugsweise steril, luftdurchlässiges Belüftungsrohr, beispielsweise eine Kanüle, wird perforierend durch den Verschluß gesteckt wird,
e) gegebenenfalls wird ein Vakuum angelegt, wobei vorzugsweise die Wirkstoff-Lösung bewegt wird,
f) abschließend, vorzugsweise nach Ablauf von ca. 12h, wird das dünne, vorzugsweise steril, luftdurchlässige Belüftungsrohn entfernt und
g) gegebenenfalls das Implantat bis zum Einsatz im vorzugsweise sterilen verschlossenen Glasgefäß von Schritt a) belassen

30. Verfahren gemäß Anspruch 29, **dadurch gekennzeichnet, daß** das Implantat von Schritt a) mindestens eine metallische durchbrochene oder geschlossene Oberfläche oder Schicht aufweist, eine keramische Beschichtung aufweist, eine polymere Beschichtung aufweist und/oder mindestens eine polymere, durchbrochene oder geschlossene Oberfläche oder Schicht aufweist

31. Verfahren gemäß einem der Ansprüche 29 oder 30, **dadurch gekennzeichnet, daß** das Implantat ein Stent, Stent Graft, Graft, Graft Verbinder, Polymeroberflächen-Stent oder Katheter ist.

32. Verfahren gemäß einem der Ansprüche 29 bis 31, **dadurch gekennzeichnet, daß** der Wirkstoff ausgewählt ist aus pharmazeutischen Wirkstoffen wie beispielsweise Immunsuppressiva oder Antibiotika, vorzugsweise ausgewählt ist aus den folgenden Wirkstoffen bzw. deren Derivaten
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1 H-pyrazolo[3,4-n]pyridin-3-yl]-pyrimidin-4-ylamine); Hydralazin; Verapamil, Diltiazem, Nifedipin, Nimodipin oder anderen Ca²⁺-Kanal-Blockern; Captopril, Enalapril, Lisinopril, Quinapril oder anderen Inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednisor oder Corticosterioden; FK 506 (Tacrolimus); 17 beta-Östraclol; Cyclosporin; Mycophenol-Säure; VEGF, VEGF-Rezeptor Aktivatoren; Tranilast; Meloxicam, Celebrex, Vioxx oder anderen COX-2 Antagonisten, Indomethacin, Diclofenac, Ibuprofen, Naproxen oder anderen COX-1-Inhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK; Interleukin-10;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavopiridol;
Actinomycin D; Rheopro/Abciximab oder Probucol
insbesondere ausgewählt ist aus
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1H-pyrazolo[3,4-n]pyridin-3-yl]-pyrimidin-4-ylamine); Captopril, Enalapril, Lisinopril, Quinapril oder anderen inhibitoren Angiotensin umwandelnder Enzyme (angiotensin converting enzyme inhibitors); Losartan, Candesartan, Irbesartan, Valsartan oder anderen Antagonisten des Angiotensin II-Rezeptors ;
(Gruppe 2:) Dexamethason, Betamethason, Prednisor oder Corticosterioden; FK 506 (Tacrolimus); VE3F, VEGF-Rezeptor Aktivatoren; Inhibitoren des Plasminogen Aktivators-1 (plasminogen activator inhibitors-1) oder Serpinen;
(Gruppe 3:) Sirolimus, Rapamycin, SDZ RAD (40-O-(2-hydroxyethyl)-Rapamycin oder anderen Rapamycin-Derivativen; PDGF-Antagonisten: Paclitaxel oder 7-Hexanoyl-Taxol; Mitoxantron; Combretastatin A4; Flavopiridol.

33. Implantat gemäß einem der Ansprüche 1 bis 24 zur Behandlung oder Prophylaxe von koronaren oder peripheren Gefäßverengungen oder -verschlüssen, insbesondere von Verengungen bzw. Stenosen oder Restenosen, vorzugsweise zur Prophylaxe von Restenose.

34. Verwendung von FK506 zur Beschichtung oder zur Herstellung eines Implantats zur Behandlung oder Prophylaxe von koronaren oder peripheren. Gefäßverengungen oder -verschlüssen, insbesondere von Verengungen bzw. Stenosen oder Restenosen, vorzugsweise zur Prophylaxe von Restenose,

35. Verwendung gemäß Anspruch 34, **dadurch gekennzeichnet, daß** das Implantat ein Stent, ein Stent Graft, ein Graft, ein Graft-Verbinder, ein Führungsdrant, ein Katheter oder eine Katheterpumpe, vorzugsweise ein Stent, ein Stent Graft, ein Graft oder ein Graft-Verbinder, insbesondere ein Stent oder ein Stent Graft bzw. ein Polymeroberflächen-Stent ist.

36. Verwendung gemäß einem der Ansprüche 34 oder 35, **dadurch gekennzeichnet, daß** das FK506 am Implantat so gebunden oder angebracht ist, daß es nach Implantierung vom implantat, vorzugsweise verzögert, freigesetzt wird.

37. Polymeroberflächen-Stent enthaltend in chemisch kovalent oder nicht-kovalent gebundener oder physikalisch fixierter Form mindestens physiologisch und/oddr pharmazeutisch wirksames FK 506.

38. Polymeroberflächen-Stent gemäß Anspruch 37, **dadurch gekennzeichnet, daß** der Wirkstoff ausgewählt ist aus pharmazeutischen Wirkstoffen wie beispielsweise Immunsuppressiva öder Antibiotika, vorzugsweise ausgewählt ist aus den folgenden Wirkstoffen bzw. deren Derivaten
(Gruppe 1:) Molsidomin, Linsidomin, Natrium-Nitroprussid, Nitroglycerin oder allgemeinen NO-Donoren; Stimulatoren der löslichen Guanylat-Cyclase (sGC/soluble guanylate cyclase), beispielsweise BAY 41-2272 (5-(Cyclopropyl-2-[1-(2-fluoro-benzyl)-1H-pyrazolo[3,4-n]pyridin-3-yl]-pyrimidin-4-ylamine); Hydralazin; Verapamil, Diltiazem, Nifedipin, Nimodipin oder anderen Ca²⁺-Kanal-Blockern ; Captopril, Enalapril, Lisinopril, Quinapril oder anderen Inhibitoren Angiotensin umwandelnder Enzyme (angiotensin converting enzyme inhibitors) ; Losartan, Candesartan, Irbesartan, Valsartan oder anderen Antagonisten des Angiotensin II-Rezeptors ;
(Gruppe 2:) Dexamethason, Betamethason, Prednison oder Corticosterioden; FK 506 (Tacrolimus); 17-beta-Östradiot: Cyclosporin ; Mycophenol-Säure ; VEGF, VEGF-Rezeptor Aktivatoren; Tranilast; Meloxicam, Celebrex, Vioxx oder anderen COX-2 Antagonisten; Indomethacin, Diclofenac, Ibuprofen, Naproxen oder anderen COX-1-lnhibitoren; Inhibitoren des Plasminogen Aktivators-1 (plasminogen activator inhibitors-1) oder Serpinen; Thrombin Inhibitoren, beispielsweise Hirudin, Hirulog, Agratroban, PPACK ; lnterleukin-10;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Cisplatin; Vinblastin; Mitoxantron; Combretastatin A4; Topotecan; Methotrexat; Flavopiridol;
Actinomycin D; Rheopro/Abciximab oder Probucol
insbesondere ausgewählt ist aus
(Gruppe 1:) Molsidomin, Linsidomin, Natrium-Nitroprussid, Nitroglycerin oder allgemeinen NO-Donoren ; Stimulatoren der löslichen Guanylat-Cyclase (sGC/soluble guanylate cyclase), beispielsweise BAY 41-2272 (5-(Cyclopropyl-2-[1-(2-fluoro-benzyl)-1H-pyrazolo[3,4-n]pyridin-3-yl]-pyrimidin-4-ylamine) ; Captopril, Enalapril, Lisinopril, Quinapril oder anderem Inhibitoren Angiotensin umwandelnder Enzyme (anglotensin converting enzyme inhibitors); Losartan, Candesartan, Irbesartan, Valsartan oder anderen Antagonisten des Angiotensin II-Rezeptors;
(Gruppe 2:) Dexamethason, Betamethason, Prednisor oder Corticosterioden; FK 506 (Tacrolimus); VEGF, VEGF-Rezeptor Aktivatoren; Inhibitoren des Plasminogen Aktivators-1 (plasminogen activator inhibitors-1) oder Serpinen;
(Gruppe 3:) Sirolimus, Rapamycin, SDZ RAD (40-O-(2-hydroxyethyl)-Rapamycin oder anderen Rapamycin-Derivativen; PDGF-Antagonisten; Paclitaxel oder 7-Hexanoyl-Taxol; Mitoxantron; Combretastatin A4; Flavopiridol;
und/oder daß der Polymeroberflächen-Stent mindestens zwei, vorzugsweise 2 oder 3 physiologisch und/oder pharmazeutisch wirksame Wirkstoffe ausgewählt aus einer der Gruppen 1 bis 3 enthält, vorzugsweise maximal einen Wirkstoff aus einer Gruppe.

39. Polymeroberflächen-Stent.gemäß Anspruch 38, **dadurch gekennzeichnet, daß** wenn der weitere Wirkstoff ausgewählt ist aus Gruppe 1, dieser vom Implantat innerhalb der ersten 24 - 72 h nach Implantation freigesetzt wird und/oder, wenn der weitere Wirkstoff ausgewählt ist aus Gruppe 2, dieser vom Implantat innerhalb der ersten 48 h - 21 Tagen nach Implantation freigesetzt wird und/oder, wenn der weitere Wirkstoff ausgewählt ist aus Gruppe 3, dieser vom Implantat innerhalb von 14 Tagen bis 3 Monaten nach Implantation freigesetzt wird.

## Claims

1. An intracavernous, preferably intravascular implant comprising FK506 in chemically covalently bound or noncovalently bound or physically immobilized form, and, optionally, at least one other active agent wherein the implant comprises a substrate which is coated with FK506 and the substrate has at least one closed or perforated layer or surface which consists of a metal or a metal alloy and is homogeneous or formed from various strands.

2. The implant as claimed in claim 1, **characterized in that** the implant is suitable for the treatment or prophylaxis of coronary or peripheral vascular constrictions or occlusions, in particular of constrictions or stenoses or restenoses, preferably for the prophylaxis of restenosis.

3. The implant as claimed in claims 1 or 2, **characterized in that** the implant has at least one closed or perforated layer or surface which consists of a polymer and is homogeneous or formed from various strands.

4. The implant as claimed in claim 3, **characterized in that** at least one polymer layer covers completely or partly a closed or perforated layer or surface which consists of a metal or a metal alloy and is homogeneous or formed from various strands, preferably an optionally lattice-like structure consisting of a metal or a metal alloy.

5. The implant as claimed in claim 3, **characterized in that** the implant has at least one closed or perforated layer or surface which consists of a metal or a metal alloy and is homogeneous or formed from various strands, and at least one closed or perforated layer or surface which consists of a polymer and is homogeneous or formed from various strands.

6. The implant as claimed in claim 5, **characterized in that** the layer or surface consisting of a metal or a metal alloy is an optionally lattice-like structure consisting of a metal or a metal alloy, and/or the layer or surface consisting of a polymer is homogeneously closed or woven and/or is water- and/or corpuscle-impermeable, and/or the sequence of layers and surfaces is from the outside to the inside metal-polymer, polymer-metal, metal-polymer-metal or polymer-metal-polymer, and/or either the layer or surface consisting of a polymer is nonchemically (covalently or noncovalently) connected to the layer or surface consisting of a metal or a metal alloy, or the layer or surface consisting of a polymer is connected by means of an adhesive to the layer or surface consisting of a metal or a metal alloy.

7. The implant as claimed in any one of claims 3 to 6, **characterized in that** the polymer is selected from Dacron; polytetrafluoroethylene (PTFE/Teflon), expandable or non-expandable; polyurethane; methacrylate polymers; hydrogel or hydrogel/polyurethane blend, preferably from polytetrafluoroethylene (PTFE), expandable or non-expandable; or polyurethane; in particular from PTFE.

8. The implant as claimed in any one of claims 1 to 7, **characterized in that** the implant is a stent, a stent graft, a graft, a graft connector, a guide wire, a catheter or a catheter pump, preferably a stent, a stent graft, a graft or a graft connector, in particular a stent or a stent graft.

9. The implant as claimed in any one of claims 1 to 8, **characterized in that** the substrate has a ceramic coating, in particular of aluminum oxide or iridium oxide, to which FK506 is bound.

10. The implant as claimed in claim 9, **characterized in that** the ceramic coating is completely or partly covered by a polymeric, preferably biodegradable, coating to which, optionally, FK506 and/or another active agent is bound or in which FK506 and/or another active agent has been dissolved before application of the coating.

11. The implant as claimed in any one of claims 1 to 10, **characterized in that** the substrate has a polymeric coating, in particular of methacrylate polymers, polyurethane, PTFE, hydrogel or hydrogel/polyurethane blend, in particular PTFE, to which FK506 is bound or in which FK506 has been dissolved before application of the coating.

12. The implant as claimed in any one of claims 1 to 11, **characterized in that** the metal of the implant has recesses which have been introduced by means of a laser and which are filled with FK506.

13. The implant as claimed in claim 12, **characterized in that** the metal provided with FK506-filled recesses or at least the recesses is/are coated with a biodegradable polymeric material, whereby, optionally, FK506 being bound to the polymeric coating, or FK506 having been dissolved in the polymeric material before application of the coating.

14. The implant as claimed in any of claims 1 to 13, **characterized in that** FK506 is present in the form of loaded nanoparticles or liposomes.

15. The implant as claimed in any one of claims 1 to 14 which can be produced by a process in which
a) a substrate having at least one closed or perforated layer or surface which consists of a metal or a metal alloy and is homogeneous or formed from various strands, which substrate is coated with a ceramic coating, in particular of aluminum oxide or iridium oxide, is employed
or
b) a substrate having at least one closed or perforated layer or surface which consists of a polymer and is homogeneous or formed from various strands is employed,
or
c) a substrate which is coated with a coating which is polymerized or polymerizing on the surface, in particular of methacrylate polymers, polyurethane, PTFE, hydrogel or hydrogel/polyurethane blend, is employed
or
d) a substrate having at least one closed or perforated layer or surface which consists of a metal or a metal alloy and is homogeneous or formed from various strands, and into which recesses have been introduced by means of a laser, which are filled with FK506, and then the substrate is coated with a biodegradable coating which is polymerized or polymerizing on the surface, is employed,
e) then the substrate according to a), b), c) or d) is brought into contact with an FK506 solution in aqueous or organic solvent, for example by sprinkling, spraying or immersing, optionally under vacuum,
f) then, optionally, the thus treated substrate is dried, preferably until the solvent from step e) is removed,
g) then, optionally, step e), optionally followed by step f), is repeated, preferably several times, in particular 1 to 5 times, and,
h) optionally, subsequently the treated substrate is rinsed one or more times with water or isotonic saline, and,
i) optionally, is subsequently dried.

16. The implant as claimed in claim 15, **characterized in that** in step e) FK506 is dissolved in alcohol, preferably in ethanol, in particular in a concentration of 0.5-5 g/l FK506 in ethanol and/or in step e) the implant is brought into contact with an FK506 solution in aqueous or organic solvent by immersing under vacuum preferably overnight, and/or steps f) and/or g) are not carried out and/or in step h) the implant is washed several times with saline and/or in step i) the implant is dried overnight.

17. The implant as claimed in claim 15, **characterized in that** in step e) the implant is introduced, preferably sterilely, into a preferably sterile vessel with a closure which can be perforated and which closes after completion of a perforation, for example into an injection vial, FK506 solution, is preferably sterilely introduced into the vessel, the latter is closed with the closure which can be perforated and which closes after completion of a perforation, a thin, preferably sterile, air-pervious ventilation tube, for example a cannula, is pushed perforatingly through the closure, a vacuum is applied and, preferably, the FK506 solution is agitated, and finally, preferably after about 12 h have elapsed, the thin, preferably sterile, air-pervious ventilation tube is removed and/or **in that** in step e) FK506 is dissolved in alcohol, preferably in ethanol, in particular in a concentration of 3.3 mg of FK506 in 1 ml of ethanol and/or **in that** the implant remains until used in the preferably sterile closed glass vessel from step e) and/or **in that** steps f) to i) are omitted.

18. The implant as claimed in any one of claims 3 to 8 which can be produced by a process in which FK506 has been dissolved in the polymerization material before the formation of at least one closed or perforated layer or surface consisting of a polymer, or of a polymeric coating of the implant.

19. The implant as claimed in any one of claims 1 to 18, **characterized in that** FK506 is released after implantation of the implant.

20. The implant as claimed in claim 19, **characterized in that** delayed release takes place.

21. The implant as claimed in claim 20, **characterized in that** FK506 is released from the implant over a period of 24 h, preferably 48 h, in particular more than 96 h, after implantation.

22. The implant as claimed in claim 20, **characterized in that** the FK506
a) is released within <48 h or
b) over at least 48 h, preferably over at least 7 days, in particular over at least 2 and up to 21 days, from the implant after implantation, or **in that**
c) the implant shows both release patterns a) and b).

23. The implant as claimed in any one of claims 1 to 22, **characterized in that** the implant comprises at least one other active agent, preferably a pharmaceutically active agent, in particular another active agent selected from the following active agents or derivatives thereof
(Group 1:) molsidomine, linsidomine, sodium nitroprusside, nitroglycerin or general NO donors; stimulators of soluble guanylate cyclase (sGC), for example BAY 41-2272 (5-(cyclopropyl-2-[1-(2-fluorobenzyl)-1H-pyrazolo[3,4-n]pyridin-3-yl]pyrimidin-4-ylamine); hydralazine, verapamil, diltiazem, nifedipine, nimodipine or other Ca²⁺ channel blockers; captopril, enalapril, lisinopril, quinapril or other inhibitors of angiotensin converting enzymes (angiotensin converting enzyme inhibitors); losartan, candesartan, irbesartan, valsartan or other antagonists of the angiotensin II receptor;
(Group 2:) dexamethasone, betamethasone, prednisone or other corticosteroids; 17-beta-estradiol; cyclosporin; mycophenolic acid; VEGF, VEGF receptor activators; tranilast; meloxicam, celebrex, vioxx or other COX-2 antagonists; indomethacin, diclofenac, ibuprofen, naproxen or other COX-1 inhibitors; inhibitors of plasminogen activator 1 (plasminogen activator inhibitors-1) or serpins; thrombin inhibitors, for example hirudin, hirulog, agratroban, PPACK; interleukin-10;
(Group 3:) sirolimus, rapamycin, SDZ RAD (40-O-(2-hydroxyethyl)rapamycin or other rapamycin derivatives; PDGF antagonists; paclitaxel or 7-hexanoyl-taxol; cisplatin; vinblastine; mitoxantrone; combretastatin A4; topotecan; methotrexate; flavopiridol;
actinomycin D; Rheopro/abciximab or probucol.

24. The implant as claimed in claim 23, **characterized in that** if the other active agent is selected from group 1 this active agent is released from the implant within the first 24 - 72 h after implantation and/or, if the other active agent is selected from group 2, the latter is released from the implant within the first 48 h - 21 days after implantation and/or, if the other active agent is selected from group 3, the latter is released from the implant within 14 days to 3 months after implantation.

25. A process for producing an implant as claimed in any of claims 1 to 22 with the following steps:
a) an implant having at least one closed or perforated layer or surface which consists of a metal or a metal alloy and is homogeneous or formed from various strands, as claimed in any of claims 1, 2, or 4 to 8, which implant is coated with a ceramic coating, in particular of aluminum oxide or irridium oxide,
or
b) an implant having at least one closed or perforated layer or surface which consists of a polymer and is homogeneous or formed from various strands, as claimed in any of claims 3 to 8,
or
c) an implant as claimed in any one of claims 1 to 8, which is coated with a coating which is polymerized or polymerizing on the surface, in particular of methacrylate polymers, polyurethane, PTFE, hydrogel or hydrogel/polyurethane blend,
or
d) an implant as claimed in any one of claims 1, 2, or 4 to 8 having at least one closed or perforated layer or surface which consists of a metal or a metal alloy and is homogeneous or formed from various strands, and into which recesses have been introduced by means of a laser, which are filled with FK506, and then the implant is coated with a preferably biodegradable coating which is polymerized or polymerizing on the surface, is employed,
e) then the implant according to a), b), c) or d) is brought into contact with an FK506 solution in aqueous or organic solvent, for example by sprinkling, spraying or immersing, optionally under vacuum,
f) then, optionally, the implant is dried, preferably until the solvent from step e) is evaporated,
g) then, optionally, step e), optionally followed by step f), is repeated, preferably several times, in particular 1 to 5 times, and,
h) optionally, subsequently the implant is rinsed one or more times with water or isotonic saline, and,
i) optionally, is subsequently dried.

26. The process as claimed in claim 25, **characterized in that** in step e) FK506 is dissolved in alcohol, preferably in ethanol, in particular in a concentration of 0.5-5 g/l FK506 in ethanol and/or **in that** in step e) the implant is brought into contact with an FK506 solution in aqueous or organic solvent by immersing under vacuum preferably overnight, and/or **in that** steps f) and/or g) are not carried out, and/or **in that** in step h) the implant is washed several times with saline, and/or **in that** in step i) the implant is dried overnight.

27. The process as claimed in claim 25, **characterized in that** in step e) the implant is introduced, preferably sterilely, into a preferably sterile vessel with a closure which can be perforated and which closes after completion of a perforation, for example into an injection vial, FK506 solution, is preferably sterilely introduced into the vessel, the latter is closed with the closure which can be perforated and which closes after completion of a perforation, a thin, preferably sterile, air-pervious ventilation tube, for example a cannula, is pushed perforatingly through the closure, a vacuum is applied and, preferably, the FK506 solution is agitated, and finally, preferably after about 12 h have elapsed, the thin, preferably sterile, air-pervious ventilation tube is removed and/or in step e) FK506 is dissolved in alcohol, preferably in ethanol, in particular in a concentration of 3.3 mg of FK506 in 1 ml of ethanol and/or **in that** the implant remains until used in the preferably sterile closed glass vessel from step e) and/or **in that** steps f) to i) are omitted.

28. The process for producing implants as claimed in any one of claims 3 to 8, in which FK506 has been dissolved in the polymerization material before the formation of at least one closed or perforated layer or surface consisting of a polymer, or of a polymeric coating of the implant.

29. A process for producing intracavernous, preferably intravascular implants coated with active agents containing at least FK506, with the following steps:
a) the implant is introduced, preferably sterilely, into a preferably sterile vessel with a closure which can be perforated and closes after completion of a perforation, for example an injection vial,
b) preferably sterile solution of the active agent, preferably in an organic solvent with a low vapor pressure, in particular in alcohol such as ethanol or methanol, is introduced into the vessel,
c) the vessel is closed with the closure which can be perforated and closes after completion of a perforation,
d) a thin, preferably sterile, air-pervious ventilation tube, for example a cannula, is pushed perforatingly through the closure,
e) optionally a vacuum is applied, whereby the active agent solution is preferably agitated,
f) finally, preferably after about 12 h have elapsed, the thin, preferably sterile, air-pervious ventilation tube is removed and,
g) optionally, the implant is left until used in the preferably sterile closed glass vessel from step a).

30. The process as claimed in claim 29, **characterized in that** the implant from step a) has at least one metallic perforated or closed surface or layer, has a ceramic coating, has a polymeric coating and/or has at least one polymeric, perforated or closed surface or layer.

31. The process as claimed in either of claims 29 or 30, **characterized in that** the implant is a stent, stent graft, graft, graft connector, polymeric surface stent or catheter.

32. The process as claimed in any one of claims 29 to 31, **characterized in that** the active agent is selected from pharmaceutical active agents such as, for example, immunosuppressants or antibiotics, is preferably selected from the following active agents or derivatives thereof
(Group 1:) molsidomine, linsidomine, sodium nitroprusside, nitroglycerin or general NO donors; stimulators of soluble guanylate cyclase (sGC), for example BAY 41-2272 (5-(cyclopropyl-2-[1-(2-fluorobenzyl)-1H-pyrazolo[3,4-n]pyridin-3-yl]pyrimidin-4-ylamine); hydralazine, verapamil, diltiazem, nifedipine, nimodipine or other Ca²⁺ channel blockers; captopril, enalapril, lisinopril, quinapril or other inhibitors of angiotensin converting enzymes (angiotensin converting enzyme inhibitors); losartan, candesartan, irbesartan, valsartan or other antagonists of the angiotensin II receptor;
(Group 2:) dexamethasone, betamethasone, prednisone or corticosteroids; FKS06 (tacrolimus); 17-beta-estradiol; cyclosporin; mycophenolic acid; VEGF, VEGF receptor activators; tranilast; meloxicam, celebrex, vioxx or other COX-2 antagonists; indomethacin, diclofenac, - ibuprofen, naproxen or other COX-1 inhibitors; inhibitors of plasminogen activator-1 (plasminogen activator inhibitors-1) or serpins; thrombin inhibitors, for example hirudin, hirulog, agratroban, PPACK; interleukin-10;
(Group 3:) sirolimus, rapamycin, SDZ RAD (40-O-(2-hydroxyethyl)rapamycin or other rapamycin derivatives; PDGF antagonists; paclitaxel or 7-hexanoyl-taxol; cisplatin; vinblastine; mitoxantrone; combretastatin A4; topotecan; methotrexate; flavopiridol;
actinomycin D; Rheopro/abciximab or probucol,
in particular is selected from
(Group 1:) molsidomine, linsidomine, sodium nitroprusside, nitroglycerin or general NO donors; stimulators of soluble guanylate cyclase (sGC), for example BAY 41-2272 (5-(cyclopropyl-2-[1-(2-fluorobenzyl)-1H-pyrazolo[3,4-n]pyridin-3-yl]pyrimidin-4-ylamine); captopril, enalapril, lisinopril, quinapril or other inhibitors of angiotensin converting enzymes (angiotensin converting enzyme inhibitors); losartan, candesartan, irbesartan, valsartan or other antagonists of the angiotensin II receptor;
(Group 2:) dexamethasone, betamethasone, prednisone or corticosteroids; FK506 (tacrolimus); VEGF, VEGF receptor activators; inhibitors of plasminogen activator 1 (plasminogen activator inhibitors-1) or serpins;
(Group 3:) sirolimus, rapamycin, SDZ RAD (40-O-(2-hydroxyethyl)rapamycin or other rapamycin derivatives; PDGF-antagonists; paclitaxel or 7-hexanoyl-taxol; mitoxantrone; combretastatin A4; flavopiridol.

33. The implant as claimed in any one of claims 1 to 24 for the treatment or prophylaxis of coronary or peripheral vascular constrictions or occlusions, in particular of constrictions or stenoses or restenoses, preferably for the prophylaxis of restenosis.

34. The use of FK506 for the coating or for the production of an implant for the treatment or prophylaxis of coronary or peripheral vascular constructions or occlusions, in particular of constrictions or stenoses or restenoses, preferably for the prophylaxis of restenosis.

35. The use as claimed in claim 34, **characterized in that** the implant is a stent, a stent graft, a graft, a graft connector, a guide wire, a catheter or a catheter pump, preferably a stent, a stent graft, a graft or a graft connector, in particular a stent or a stent graft or a polymeric surface stent.

36. The use as claimed in either of claims 34 or 35, **characterized in that** the FK506 is bound or attached to the implant in such a way that it is released, preferably in a delayed manner, from the implant after implantation.

37. A polymeric surface stent comprising at least physiologically and/or pharmaceutically active FK506 in chemically covalently bound or noncovalently bound or physically immobilized form.

38. The polymeric surface stent as claimed in claim 37, **characterized in that** the active agent is selected from pharmaceutically active agents such as, for example, immunosuppressants or antibiotics, is preferably selected from the following active agents or derivatives thereof
(Group 1:) molsidomine, linsidomine, sodium nitroprusside, nitroglycerin or general NO donors; stimulators of soluble guanylate cyclase (sGC), for example BAY 41-2272 (5-(cyclopropyl-2-[1-(2-fluorobenzyl)-1H-pyrazolo[3,4-n]pyridin-3-yl]pyrimidin-4-ylamine); hydralazine, verapamil, diltiazem, nifedipine, nimodipine or other Ca²⁺ channel blockers; captopril, enalapril, lisinopril, quinapril or other inhibitors of angiotensin converting enzymes (angiotensin converting enzyme inhibitors); losartan, candesartan, irbesartan, valsartan or other antagonists of the angiotensin II receptor;
(Group 2:) dexamethasone, betamethasone, prednisone or corticosteroids; FK506 (tacrolimus); 17-beta-estradiol; cyclosporin; mycophenolic acid; VEGF, VEGF receptor activators; tranilast; meloxicam, celebrex, vioxx or other COX-2 antagonists; indomethacin, diclofenac, ibuprofen, naproxen or other COX-1 inhibitors; inhibitors of plasminogen activator-1 (plasminogen activator inhibitors-1) or serpins; thrombin inhibitors, for example hirudin, hirulog, agratroban, PPACK; interleukin-10;
(Group 3:) sirolimus, rapamycin, SDZ RAD (40-O-(2-hydroxyethyl)rapamycin or other rapamycin derivatives; PDGF antagonists; paclitaxel or 7-hexanoyl-taxol; cisplatin; vinblastine; mitoxantrone; combretastatin A4; topotecan; methotrexate; flavopiridol;
actinomycin D; Rheopro/abciximab or probucol,
in particular is selected from
(Group 1:) molsidomine, linsidomine, sodium nitroprusside, nitroglycerin or general NO donors; stimulators of soluble guanylate cyclase (sGC), for example BAY 41-2272 (5-(cyclopropyl-2-[1-(2-fluorobenzyl)-1H-pyrazolo[3,4-n]pyridin-3-yl]pyrimidin-4-ylamine); captopril, enalapril, lisinopril, quinapril or other inhibitors of angiotensin converting enzymes (angiotensin converting enzyme inhibitors); losartan, candesartan, irbesartan, valsartan or other antagonists of the angiotensin II receptor;
(Group 2:) dexamethasone, betamethasone, prednisone or corticosteroids; FK506 (tacrolimus); VEGF, VEGF receptor activators; inhibitors of plasminogen activator-1 (plasminogen activator inhibitors-1) or serpins;
(Group 3:) sirolimus, rapamycin, SDZ RAD (40-O-(2-hydroxyethyl)rapamycin or other rapamycin derivatives; PDGF-antagonists; paclitaxel or 7-hexanoyl-taxol; mitoxantrone; combretastatin A4; flavopiridol;
and/or **in that** the polymeric surface stent comprises at least two, preferably 2 or 3, physiologically and/or pharmaceutically active active agents selected from one of groups 1 to 3, preferably a maximum of one active agent from one group.

39. The polymeric surface stent as claimed in claim 38, **characterized in that** if the other active agent is selected from group 1 this active agent is released from the implant within the first 24-72 h after implantation, and/or, if the other active agent is selected from group 2, this active agent is released from the implant within the first 48 h-21 days after implantation, and/or, if the other active agent is selected from group 3, this active agent is released from the implant within 14 days to 3 months after implantation.

## Revendications

1. Implant intracaverneux, de préférence, intravasculaire contenant sous une forme liée chimiquement de manière covalente ou de manière non covalente ou fixée physiquement, le FK506 et éventuellement au moins un autre principe actif, l'implant présentant un substrat qui est revêtu de FK506 et le substrat présentant au moins une couche ou une surface fermée ou perforée, homogène ou formée de divers brins, constituée d'au moins un métal ou un alliage métallique.

2. Implant selon la revendication 1, **caractérisé en ce que** l'implant est approprié pour le traitement ou la prophylaxie des rétrécissements ou obturations vasculaires coronaires ou périphériques, en particulier des rétrécissements ou des sténoses ou des resténoses, de préférence, en prophylaxie de la resténose.

3. Implant selon l'une des revendications 1 ou 2, **caractérisé en ce que** l'implant présente au moins une couche ou une surface fermée ou perforée, homogène, ou formée de divers brins, constituée d'un polymère.

4. Implant selon la revendication 3, **caractérisé en ce qu'**au moins une couche de polymère recouvre totalement ou partiellement une couche ou une surface fermée ou perforée, homogène ou formée de divers brins, constituée d'un métal ou d'un alliage métallique, de préférence une structure constituée de métal ou d'un alliage métallique éventuellement sous forme de grillage.

5. Implant selon la revendication 3, **caractérisé en ce que** l'implant présente au moins une couche ou une surface fermée ou perforée, homogène ou formée de divers brins, constituée d'un métal ou d'un alliage métallique, et au moins une couche ou une surface fermée ou perforée, homogène ou formée de divers brins, constituée d'un polymère.

6. Implant selon la revendication 5, **caractérisé en ce que** la couche ou surface constituée d'un métal ou d'un alliage métallique est une structure constituée d'un métal ou d'un alliage métallique éventuellement sous forme de grillage et/ou la couche ou surface constituée d'un polymère est fermée de façon homogène ou tissée et/ou est imperméable à l'eau et/ou aux corpuscules et/ou la série des couches et des surfaces de l'extérieur vers l'intérieur est de métal-polymère, de polymère-métal, de métal-polymère-métal ou de polymère-métal-polymère et/ou la couche ou la surface constituée d'un polymère n'est pas liée chimiquement (de manière covalente ou non covalente) avec la couche ou surface constituée d'un métal ou d'un alliage métallique ou la couche ou surface constituée d'un polymère est liée au moyen d'un adhésif avec la couche ou surface constituée d'un métal ou d'un alliage métallique.

7. Implant selon l'une quelconque des revendications 3 à 6, **caractérisé en ce que** le polymère est choisi parmi le dacron; le polytétrafluoro-éthylène (PTFE/téflon) dilatable ou non dilatable ; le polyuréthane ; les polymères méthacrylates ; l'hydrogel ou le mélange hydrogel/polyuréthane, de préférence le polytétrafluoroéthylène (PTFE) dilatable ou non dilatable ; ou le polyurétane ; en particulier le PTFE.

8. Implant selon l'une quelconque des revendications 1 à 7, **caractérisé en ce que** l'implant est un stent, une endoprothèse, un greffon, un élément de liaison de greffon, un fil de guidage, un cathéter ou une pompe de cathéter, de préférence un stent, une endoprothèse, un greffon ou un élément de liaison de greffon, en particulier un stent ou une endoprothèse.

9. Implant selon l'une quelconque des revendications 1 à 8, **caractérisé en ce que** le substrat présente un revêtement céramique, en particulier constitué d'oxyde d'aluminium ou d'oxyde d'iridium, auquel le FK506est lié.

10. Implant selon la revendication 9, **caractérisé en ce que** le revêtement céramique est recouvert complètement ou partiellement d'un revêtement polymère de préférence biodégradable auquel le FK506 et/ou un autre principe actif est éventuellement lié ou dans lequel le FK506 et/ou un autre principe actif a été dissous avant application du revêtement.

11. Implant selon l'une quelconque des revendications 1 à 10, **caractérisé en ce que** le substrat présente un revêtement polymère, en particulier en polymères méthacrylates, en polyuréthane, PTFE, hydrogel ou mélange d'hydrogel/polyuréthane, en particulier en PTFE, auquel le FK506 est lié ou dans lequel le FK506 a été dissous avant l'application du revêtement.

12. Implant selon l'une quelconque des revendications 1 à 11, **caractérisé en ce que** le métal de l'implant présente des cavités aménagées par laser qui sont remplies de FK506.

13. Implant selon la revendication 12, **caractérisé en ce que** le métal pourvu de cavités remplies de FK506 ou au moins les cavités est recouvert/ sont recouvertes d'un matériau polymère biodégradable, le FK506 étant éventuellement lié au revêtement polymère ou le FK506 étant dissous dans le matériau polymère avant application du revêtement.

14. Implant selon l'une quelconque des revendications 1 à 13, **caractérisé en ce que** le FK506 se présente sous forme de nanoparticules ou de liposomes chargés.

15. Implant selon l'une quelconque des revendications 1 à 14, pouvant être produit par un procédé dans lequel
a) un substrat présentant au moins une couche ou surface fermée ou perforée, homogène ou formée de divers brins, constituée d'un métal ou d'un alliage métallique, qui est revêtu d'un revêtement céramique, en particulier en oxyde d'aluminium ou en oxyde d'iridium, est utilisé,
ou
b) un substrat présentant au moins une couche ou surface fermée ou perforée, homogène ou formée de divers brins, constituée d'un polymère, est utilisé, ou
c) un substrat qui est revêtu d'un revêtement polymérisé ou polymérisant sur la surface, en particulier en polymères méthacrylates, polyuréthane, PTFE, hydrogel ou un mélange d'hydrogel/polyuréthane, est utilisé,
ou
d) un substrat comportant au moins une couche ou surface fermée ou perforée, homogène ou formée de plusieurs brins, constituée d'un métal ou d'un alliage métallique, dans laquelle des cavités qui sont remplies de FK 506 sont aménagées au moyen d'un laser, et ensuite, le substrat est revêtu d'un revêtement biodégradable polymérisé ou polymérisant sur la surface, est utilisé,
e) ensuite, le substrat selon les points a), b), c) ou d) est mis en contact avec une solution de FK506 dans un solvant aqueux ou organique, par exemple par imprégnation, pulvérisation ou immersion éventuellement sous vide,
f) ensuite éventuellement, le substrat ainsi traité est séché, de préférence jusqu'à élimination du solvant de l'étape e),
g) puis éventuellement l'étape e) éventuellement suivie de l'étape f) est répétée, de préférence à plusieurs reprises, en particulier 1 à 5 fois, et
h) éventuellement ensuite, le substrat traité est rincé une ou plusieurs fois à l'eau ou avec une solution physiologique isotonique, et
i) est ensuite éventuellement séché.

16. Implant selon la revendication 15, **caractérisé en ce que** à l'étape e), le FK506 est dissous dans l'alcool, de préférence dans l'éthanol, en particulier en une concentration de 0,5 à 5 g/l de FK506 dans de l'éthanol, et/ou **en ce que** à l'étape e), l'implant est mis en contact sous vide de préférence pendant la nuit, par immersion avec une solution de FK506 dans un solvant aqueux ou organique et/ou les étapes f) et/ou g) ne sont pas réalisées et/ou à l'étape h), l'implant est lavé à plusieurs reprises avec une solution physiologique et/ou à l'étape i), l'implant est séché pendant la nuit.

17. Implant selon la revendication 15, **caractérisé en ce que** à l'étape e), l'implant de préférence stérile est placé de préférence dans un récipient stérile avec un dispositif de fermeture perforable se fermant après la fin d'une perforation, par exemple dans un flacon pour injection, une solution de FK506 est versée dans le récipient, de préférence de façon stérile, celui-ci est fermé avec le dispositif de fermeture perforable se fermant après la fin d'une perforation, qu'un tube d'aération fin, de préférence stérile, perméable à l'air, par exemple une canule est inséré de manière perforante au travers du dispositif de fermeture, qu'un vide est créé et de préférence, que la solution de FK506 est agitée et ensuite, de préférence après écoulement d'environ 12 h, le tube d'aération fin, de préférence stérile perméable à l'air est retiré et/ou qu'à l'étape e), le FK506 est dilué dans l'alcool, de préférence dans l'éthanol, en particulier en une concentration de 3,3 mg de FK506 dans 1 ml d'éthanol et/ou que l'implant reste de préférence jusqu'à l'utilisation dans le récipient en verre stérile fermé de l'étape e) et/ou que les étapes f) à i) ne sont pas réalisées.

18. Implant selon l'une quelconque des revendications 3 à 8, pouvant être produit par un procédé dans lequel avant la formation d'au moins une couche ou surface fermée ou perforée constituée d'un polymère ou du revêtement polymère de l'implant, le FK506 a été dissous dans le matériau de polymérisation.

19. Implant selon l'une quelconque des revendications 1 à 18, **caractérisé en ce que**, après implantation de l'implant, le FK506 est libéré.

20. Implant selon la revendication 19, **caractérisé en ce que** la libération a lieu de façon retardée.

21. Implant selon la revendication 20, **caractérisé en ce que** le FK506 est libéré de l'implant sur une durée de 24 h, de préférence de 48 h, en particulier de plus de 96 h après implantation.

22. Implant selon la revendication 20, **caractérisé en ce que** le FK506 est libéré de l'implant après implantation
a) en moins de 48 h, ou
b) sur une durée d'au moins 48 h, de préférence d'au moins 7 jours, en particulier sur au moins 2 à 21 jours, ou que
c) l'implant présente les deux schémas de libération a) et b).

23. Implant selon l'une quelconque des revendications 1 à 22, **caractérisé en ce que** l'implant contient au moins un autre principe actif, de préférence un principe actif pharmaceutique, en particulier un autre principe actif choisi parmi les principes actifs suivants ou leurs dérivés :
(groupe 1) : molsidomine, linsidomine, nitroprusside de sodium, nitroglycérine ou donneurs de NO généraux ; stimulateurs de la guanylate-cyclase soluble (sGC/soluble guanylate cyclase), par exemple BAY 41-2272 (5-(cyclopropyl-2-[1-(2-fluorobenzyl)-1 H -pyrazolo[3,4-n]pyridin-3-yl]-pyrimidin-4-ylamine); hydralazine ; verapamil, diltiazem, nifedipine, nimodipine ou autres inhibiteurs du canal calcique ; captopril, énalapril, lisinopril, quinapril ou autres inhibiteurs d'enzymes de conversion de l'angiotensine (angiotensin converting enzyme inhibitors) ; losartan, candesartan, irbesartan, valsartan ou autres antagonistes du récepteur de l'angiotensine II ;
(groupe 2) : dexaméthasone, bétaméthasone, prednisone ou autres corticostéroïdes; 17-bêta-oestradiol ; cyclosporine; acide mycophénolique; VEGF, activateurs du récepteur de VEGF ; tranilast ; meloxicam, celebrex, vioxx ou autres antagonistes de COX-2 ; indométhacine, diclofénac, ibuprofène, naproxène ou autres inhibiteurs de COX-1 ; inhibiteurs de l'activateur-1 du plasminogène (plasminogen activator inhibitors-1) ou serpines ; inhibiteurs de la thrombine, par exemple hirudine, hirulog, agratroban, PPACK ; interleukine-10 ;
(groupe 3) : sirolimus, rapamycine, SDZ RAD (40-O-(2-hydroxyéthyl)-rapamycine ou autres dérivés de rapamycine ; antagonistes de PDGF ; paclitaxel ou 7-hexanoyl-taxol ; cisplatine ; vinblastine ; mitoxantrone ; combretastatine A4 ; topotecan ; méthotrexate ; flavopiridol ;
actinomycine D ; rhéopro/abciximab ou probucol.

24. Implant selon la revendication 23, **caractérisé en ce que** si l'autre principe actif est choisi dans le groupe 1, celui-ci est libéré de l'implant dans les premières 24 à 72 heures après implantation et/ou si l'autre principe actif est choisi dans le groupe 2, celui-ci est libéré de l'implant dans les premières 48 h à 21 jours après implantation et/ou si l'autre principe actif est choisi dans le groupe 3, celui-ci est libéré de l'implant dans les 14 jours à 3 mois après implantation.

25. Procédé de fabrication d'un implant selon l'une quelconque des revendications 1 à 22 comprenant les étapes suivantes :
a) un implant présantant au moins une couche ou surface fermée ou perforée, homogène ou formée de divers brins, constituée de métal ou d'un alliage métallique, selon l'une quelconque des revendications 1, 2 ou 4 à 8, qui est revêtu d'un revêtement céramique, en particulier en oxyde d'aluminium ou en oxyde d'iridium,
ou
b) un implant présantant au moins une couche ou surface fermée ou perforée, homogène ou formée de divers brins, constituée d'un polymère, selon l'une quelconque des revendications 3 à 8,
ou
c) un implant selon l'une quelconque des revendications 1 à 8, qui est revêtu d'un revêtement polymérisé ou polymérisant sur la surface, en particulier en polymères méthacrylates, polyuréthane, PTFE, hydrogel ou mélange d'hydrogel/polyuréthane,
ou
d) un implant selon l'une quelconque des revendications 1, 2 ou 4 à 8, comportant au moins une couche ou surface fermée ou perforée, homogène ou formée de divers brins, constituée de métal ou d'un alliage métallique, dans laquelle des cavités sont aménagées par laser, qui sont remplies de FK506 et ensuite l'implant est revêtu d'un revêtement polymérisé ou polymérisant sur la surface, de préférence biodégradable, est utilisé,
e) ensuite l'implant selon les points a), b) c) ou d) est mis en contact avec une solution de FK506 dans un solvant aqueux ou organique, par exemple par imprégnation, pulvérisation ou immersion, éventuellement sous vide,
f) ensuite l'implant est éventuellement séché, de préférence jusqu'à ce que le solvant de l'étape e) soit évaporé,
g) puis éventuellement l'étape e) éventuellement suivie de l'étape f) est répétée, de préférence plusieurs fois, en particulier 1 à 5 fois et
h) ensuite éventuellement, l'implant est rincé une ou plusieurs fois à l'eau ou avec une solution physiologique isotonique et
i) éventuellement, il est ensuite séché.

26. Procédé selon la revendication 25, **caractérisé en ce que** à l'étape e), le FK506 est dissous dans l'alcool, de préférence dans l'éthanol, en particulier en une concentration de 0,5 à 5 g/l de FK506 dans de l'éthanol, et/ou **en ce que** à l'étape e), l'implant est mis en contact sous vide de préférence pendant la nuit, par immersion avec une solution de FK506 dans un solvant aqueux ou organique et/ou les étapes f) et/ou g) ne sont pas réalisées et/ou à l'étape h), l'implant est lavé à plusieurs reprises avec une solution physiologique et/ou à l'étape i), l'implant est séché pendant la nuit.

27. Procédé selon la revendication 25, **caractérisé en ce que** à l'étape e), l'implant de préférence stérile est placé de préférence dans un récipient stérile avec un dispositif de fermeture perforable se fermant après la fin d'une perforation, par exemple dans un flacon pour injection, une solution de FK506 est versée dans le récipient, de préférence de façon stérile, celui-ci est fermé avec le dispositif de fermeture perforable se fermant après la fin d'une perforation, qu'un tube d'aération fin, de préférence stérile, perméable à l'air, par exemple une canule est inséré de manière perforante au travers du dispositif de fermeture, qu'un vide est créé et de préférence, que la solution de FK506 est maintenue en mouvement et ensuite, de préférence après écoulement d'environ 12 h, le tube d'aération fin de préférence stérile perméable à l'air est retiré et/ou qu'à l'étape e), le FK506 est dilué dans l'alcool, de préférence dans l'éthanol, en particulier en une concentration de 3,3 mg de FK506 dans 1 ml d'éthanol et/ou que l'implant reste de préférence jusqu'à l'utilisation dans le récipient en verre stérile fermé de l'étape e) et/ou que les étapes f) à i) ne sont pas réalisées.

28. Procédé de fabrication d'implants selon l'une quelconque des revendications 3 à 8, dans lequel avant la formation d'au moins une couche ou surface constituée d'un polymère, fermée ou perforée ou d'un revêtement polymère de l'implant, le FK506 a été dilué dans le matériau de polymérisation.

29. Procédé de fabrication d'implants intracaverneux, de préférence intravasculaires revêtus de principes actifs contenant au moins le FK506, comprenant les étapes suivantes:
a) l'implant est placé de préférence de façon stérile dans un récipient de préférence stérile doté d'un dispositif de fermeture perforable se fermant après la fin d'une perforation, par exemple un flacon pour injection,
b) de préférence une solution de principe actif stérile, de préférence dans un solvant organique ayant une pression de vapeur faible, en particulier dans de l'alcool tel que l'éthanol ou le méthanol est placée dans le récipient,
c) le récipient est fermé avec le dispositif de fermeture perforable se fermant après la fin d'une perforation,
d) un tube d'aération fin, de préférence stérile, perméable à l'air, par exemple une canule, est inséré de manière perforante dans le dispositif de fermeture ;
e) éventuellement un vide est créé, la solution de principe actif étant de préférence agitée,
f) enfin, de préférence après écoulement d'environ 12 h, le tube d'aération fin, de préférence stérile, perméable à l'air est retiré, et
g) éventuellement l'implant est laissé jusqu'à l'utilisation dans le récipient en verre fermé de préférence de façon stérile de l'étape a).

30. Procédé selon la revendication 29, **caractérisé en ce que** l'implant de l'étape a) présente au moins une surface ou une couche métallique percée ou fermée, présente un revêtement céramique, présente un revêtement polymère et/ou présente au moins une surface ou couche polymère perforée ou fermée.

31. Procédé selon l'une quelconque des revendications 29 ou 30, **caractérisé en ce que** l'implant est un stent, une endoprothèse, un greffon, un élément de liaison de greffon, un stent à surface polymère ou un cathéter.

32. Procédé selon l'une quelconque des revendications 29 à 31, **caractérisé en ce que** le principe actif est choisi parmi des principes actifs pharmaceutiques comme par exemple les immunosuppresseurs ou les antibiotiques, de préférence parmi les principes actifs suivants ou leurs dérivés :
(groupe 1) : molsidomine, linsidomine, nitroprusside de sodium, nitroglycérine ou donneurs de NO généraux; stimulateurs de la guanylate-cyclase soluble (sGC/soluble guanylate cyclase), par exemple BAY 41-2272 (5-(cyclopropyl-2-[1-(2-fluorobenzyl)-1 H-pyrazolo[3,4-n]pyridin-3-yl]-pyrimidin-4-ylamine) ; hydralazine ; verapamil, diltiazem, nifedipine, nimodipine ou autres inhibiteurs du canal calcique ; captopril, énalapril, lisinopril, quinapril ou autres inhibiteurs d'enzymes de conversion de l'angiotensine (angiotensin converting enzyme inhibitors) ; losartan, candesartan, irbesartan, valsartan ou autres antagonistes du récepteur de l'angiotensine II ;
(groupe 2) : dexaméthasone, bétaméthasone, prednisone ou corticostéroïdes ; FK 506 (tacrolimus) ; 17-bêta-oestradiol ; cyclosporine; acide mycophénolique ; VEGF, activateurs du récepteur de VEGF ; tranilast ; meloxicam, celebrex, vioxx ou autres antagonistes de COX-2 ; indométhacine, diclofénac, ibuprofène, naproxène ou autres inhibiteurs de COX-1 ; inhibiteurs de l'activateur-1 du plasminogène (plasminogen activator inhibitors-1) ou serpines ; inhibiteurs de la thrombine, par exemple hirudine, hirulog, agratroban, PPACK ; interleukine 10 ;
(groupe 3) : sirolimus, rapamycine, SDZ RAD (40-O-(2-hydroxyéthyl)-rapamycine ou autres dérivés de rapamycine ; antagonistes de PDGF ; paclitaxel ou 7-hexanoyl-taxol ; cisplatine ; vinblastine ; mitoxantrone ; combretastatine A4 ; topotecan; méthotrexate; flavopiridol;
actinomycine D ; rhéopro/abciximab ou probucol,
en particulier choisi parmi
(groupe 1) : molsidomine, linsidomine, nitroprusside de sodium, nitroglycérine ou donneurs de NO généraux ; stimulateurs de la guanylate-cyclase soluble (sGC/soluble guanylate cyclase), par exemple BAY 41-2272 (5-(cyclopropyl-2-[1-(2-fluorobenzyl)-1H-pyrazolo[3,4-n]pyridin-3-yl]-pyrimidin-4-ylamine) ; captopril, énalapril, lisinopril, quinapril ou autres inhibiteurs d'enzymes de conversion de l'angiotensine (angiotensin converting enzyme inhibitors) ; losartan, candesartan, irbesartan, valsartan ou autres antagonistes du récepteur de l'angiotensine II ;
(groupe 2) : dexaméthasone, bétaméthasone, prednisone ou corticostéroïdes ; FK 506 (tacrolimus) ; VEGF, activateurs du récepteur de VEGF ; inhibiteurs de l'activateur-1 du plasminogène (plasminogen activator inhibitors-1), ou serpines ;
(groupe 3) : sirolimus, rapamycine, SDZ RAD (40-O-(2-hydroxyéthyl)-rapamycine ou autres dérivés de rapamycine ; antagonistes de PDGF ; paclitaxel ou 7-hexanoyl-taxol ; mitoxantrone ; combretastatine A4 ; flavopiridol.

33. Implant selon l'une quelconque des revendications 1 à 24 pour le traitement ou la prophylaxie des rétrécissements ou obturations vasculaires coronaires ou périphériques, en particulier des rétrécissements ou des sténoses ou des resténoses, de préférence, pour la prophylaxie de la resténose.

34. Utilisation de FK506 pour le revêtement ou pour là fabrication d'un implant destiné au traitement ou à la prophylaxie des rétrécissements ou obturations vasculaires coronaires ou périphériques, en particulier des rétrécissements ou des sténoses ou des resténoses, de préférence, pour la prophylaxie de la resténose.

35. Utilisation selon la revendication 34, **caractérisée en ce que** l'implant est un stent, une endoprothèse, un greffon, un élément de liaison de greffon, un fil de guidage, un cathéter ou une pompe de cathéter, de préférence un stent, une endoprothèse, un greffon ou un élément de liaison de greffon, en particulier un stent ou une endoprothèse ou un stent à surface polymère.

36. Utilisation selon l'une quelconque des revendications 34 ou 35, **caractérisé en ce que** le FK506 est lié à l'implant ou placé sur celui-ci de telle sorte qu'après implantation de l'implant, il soit libéré de façon retardée.

37. Stent à surface polymère contenant sous une forme liée chimiquement de manière covalente ou non covalente, ou une forme physiquement fixée au moins FK506 efficace d'un point de vue physiologique et/ou pharmaceutique.

38. Stent à surface polymère selon la revendication 37, **caractérisé en ce que** le principe actif est choisi parmi les principes actifs pharmaceutiques tels que par exemple les immunosuppresseurs ou les antibiotiques, de préférence parmi les principes actifs suivantsou leurs dérivés
(groupe 1) : molsidomine, linsidomine, nitroprusside de sodium, nitroglycérine ou donneurs de NO généraux; stimulateurs de la guanylate-cyclase soluble (sGC/soluble guanylate cyclase), par exemple BAY 41-2272 (5-(cyclopropyl-2-[1-(2-fluorobenzyl)-1H-pyrazolo[3,4-n]pyridin-3-yl]-pyrimidin-4-ylamine) ; hydralazine ; verapamil, diltiazem, nifedipine, nimodipine ou autres inhibiteurs du canal calcique ; captopril, énalapril, lisinopril, quinapril ou autres inhibiteurs d'enzymes de conversion de l'angiotensine (angiotensin converting enzyme inhibitors) ; losartan, candesartan, irbesartan, valsartan ou autres antagonistes du récepteur de l'angiotensine II;
(groupe 2) : dexaméthasone, bétaméthasone, prednisone ou corticostéroïdes ; FK 506 (tacrolimus) ; 17-bêta-oestradiol ; cyclosporine; acide mycophénolique ; VEGF, activateurs du récepteur de VEGF ; tranilast ; meloxicam, celebrex, vioxx ou autres antagonistes de COX-2 ; indométhacine, diclofénac, ibuprofène, naproxène ou autres inhibiteurs de COX-1; inhibiteurs de l'activateur du plasminogène (plasminogen activator inhibitors-1) ou serpines ; inhibiteurs de la thrombine, par exemple hirudine, hirulog, agratroban, PPACK ; interleukine 10 ;
(groupe 3) : sirolimus, rapamycine, SDZ RAD (40-O-(2-hydroxyéthyl)-rapamycine ou autres dérivés de rapamycine ; antagonistes de PDGF ; paclitaxel ou 7-hexanoyl-taxol ; cisplatine ; vinblastine ; mitoxantrone ; combretastatine A4 ; topotecan ; méthotrexate ; flavopiridol ;
actinomycine D ; rhéopro/abciximab ou probucol,
en particulier choisi parmi
(groupe 1) : molsidomine, linsidomine, nitroprusside de sodium, nitroglycérine ou donneurs de NO généraux ; stimulateurs de la guanylate-cyclase soluble (sGC/soluble guanylate cyclase), par exemple BAY 41-2272 (5-(cyclopropyl-2-[1-(2-fluorobenzyl)-1H-pyrazolo[3,4-n]pyridin-3-yl]-pyrimidin-4-ylamine) ; captopril, énalapril, lisinopril, quinapril ou autres inhibiteurs d'enzymes de conversion de l'angiotensine (angiotensin converting enzyme inhibitors) ; losartan, candesartan, irbesartan, valsartan ou autres antagonistes du récepteur de l'angiotensine II ;
(groupe 2) : dexaméthasone, bétaméthasone, prednisone ou corticostéroïdes ; FK 506 (tacrolimus) ; VEGF, activateurs du récepteur de VEGF ; inhibiteurs de l'activateur-1 du plasminogène (plasminogen activator inhibitors-1) ou serpines ;
(groupe 3) : sirolimus, rapamycine, SDZ RAD (40-O-(2-hydroxyéthyl)-rapamycine ou autres dérivés de rapamycine ; antagonistes de PDGF ; paclitaxel ou 7-hexanoyl-taxol ; mitoxantrone ; combretastatine A4 ; flavopiridol; et/ou le stent à surface polymère présente, au moins deux, de préférence 2 ou 3 principes actifs efficaces d'un point de vue physiologique et/ou pharmaceutique choisis dans les groupes 1 à 3, de préférence au maximum un principe actif d'un groupe.

39. Stent à surface polymère selon la revendication 38, **caractérisé en ce que** si l'autre principe actif est choisi dans le groupe 1, celui-ci est libéré de l'implant dans les premières 24 à 72 heures après implantation et/ou si l'autre principe actif est choisi dans le groupe 2, celui-ci est libéré de l'implant dans les premières 48 h à 21 jours après implantation et/ou si l'autre principe actif est choisi dans le groupe 3, celui-ci est libéré de l'implant dans les 14 jours à 3 mois après implantation.
